# EUROPEAN PATENT SPECIFICATION

(11) **EP 2 085 475 B1**
(45) Date of publication and mention of the grant of the patent: **09.02.2011**
(21) Application number: 07830399.7
(22) Date of filing: 23.10.2007
(51) Int. Cl.: C12N 15/09, C12N 5/10, C07K 14/035, C07K 19/00

(54) **METHOD FOR PRODUCTION OF CELL STRAIN CAPABLE OF TETRACYCLINE-INDUCED GENE EXPRESSION OR CELL STRAIN HAVING CONDITIONAL GENE KNOCKOUT, AND USE OF THE CELL STRAINS**
VERFAHREN ZUR HERSTELLUNG EINES ZELLSTAMMS MIT FÄHIGKEIT ZUR TETRACYCLIN-INDUZIERTEN GENEXPRESSION ODER EINES ZELLSTAMMS MIT KONDITIONALEM GEN-KNOCKOUT SOWIE VERWENDUNG DER ZELLSTÄMME
PROCEDE DE PRODUCTION DE SOUCHE CELLULAIRE POUVANT EXPRIMER UN GENE INDUIT PAR LA TETRACYCLINE OU DE SOUCHE CELLULAIRE PRESENTANT UNE INACTIVATION CONDITIONNELLE DE GENES, ET UTILISATION DES SOUCHES CELLULAIRES

(30) Priority: 23.10.2006 JP 2006288020
(43) Date of publication of application: 05.08.2009
(73) Proprietor: Research Organization of Information and Systems, Tachikawa-shi Tokyo 190-0014 (JP)
(72) Inventor: SHIBAHARA, Keiichi, c/o National Institute of Genetics, Mishima-shi, Shizuoka 411-8540 (JP); ONO, Tatsuya, Takasaki-shi, Gunma 370-0851 (JP); NISHIJIMA, Hitoshi, c/o National Institute of Genetics, Mishima-shi, Shizuoka 411-8540 (JP)
(74) Representative: Woods, Geoffrey Corlett
(86) International application number: PCT/JP2007/070665
(87) International publication number: WO 2008/050774

(56) References cited:
- WO-A1-99/25856
- JP-A- 10 113 178
- JP-A- 11 506 901
- SHAIKH SHAMIM ET AL: "Optimization of the Tet-On system for inducible expression of RAGE." JOURNAL OF BIOMOLECULAR TECHNIQUES : JBT SEP 2006, vol. 17, no. 4, September 2006 (2006-09), pages 283-292, XP002547742 ISSN: 1524-0215
- CHAMBARD JEAN-CLAUDE AND PHILIPPE POGNONEC: "A reliable way of obtaining stable inducible clones" NUCLEIC ACIDS RESEARCH, vol. 26, no. 14, 15 July 1998 (1998-07-15), pages 3443-3444, XP002547743 ISSN: 0305-1048
- KIRCHHOFF S ET AL: "Identification of mammalian cell clones exhibiting highly regulated expression from inducible promoters" TRENDS IN GENETICS, ELSEVIER SCIENCE PUBLISHERS B.V. AMSTERDAM, NL, vol. 11, no. 6, 1 June 1995 (1995-06-01), pages 219-220, XP004207350 ISSN: 0168-9525
- YAMAZOE MITSUYOSHI ET AL: "Reverse genetic studies of the DNA damage response in the chicken B lymphocyte line DT40" DNA REPAIR, vol. 3, no. 8-9, August 2004 (2004-08), pages 1175-1185, XP002547785 ISSN: 1568-7864
- ONO TATSUYA ET AL: "Generation of tetracycline-inducible conditional gene knockout cells in a human Nalm-6 cell line" JOURNAL OF BIOTECHNOLOGY, vol. 141, no. 1-2, April 2009 (2009-04), pages 1-7, XP026049495 ISSN: 0168-1656
- HOFMANN A. ET AL.: 'Rapid retroviral delivery of tetracycline-inducible genes in a single autoregulatory cassette' PROC. NATL. ACAD. SCI. vol. 93, 1996, pages 5185 - 5190, XP002015266

### SEQUENCE LISTING

<110> RESEARCH ORGANIZATION OF INFORMATION AND SYSTEMS
<120> Method for producing of Tc-inducible gene expressing cell and conditional gene knockout cell and use thereof
<130> TF07035-01
<150> JP 06/288020
   <151> 2006-10-23
<160> 14
<170> PatentIn version 3.1
<210> 1
   <211> 60
   <212> DNA
   <213> Human cytomegalovirus
<400> 1
   taggcgtgta cggtgggagg cctatataag cagagctcgt ttagtgaacc gtcagatcgc 60
<210> 2
   <211> 36
   <212> DNA
   <213> Artificial
<220>
   <223> tet operator
<400> 2
   cgagtttact ccctatcagt gatagagaac gtatgt 36
<210> 3
   <211> 250
   <212> DNA
   <213> Artificial
<220>
   <223> tet operator sequence concatemer
<400> 3
   cgagtttact ccctatcagt gatagagaac gtatgtcgag tttactccct atcagtgata      60
   gagaacgatg tcgagtttac tccctatcag tgatagagaa cgtatgtcga gtttactccc     120
   tatcagtgat agagaacgta tgtcgagttt actccctatc agtgatagag aacgtatgtc     180
   gagtttatcc ctatcagtga tagagaacgt atgtcgagtt tactccctat cagtgataga     240
   gaacgtatgt                                                            250
<210> 4
   <211> 576
   <212> DNA
   <213> Encephalomyocarditis virus
<400> 4
   cgcccctctc cctccccccc ccctaacgtt actggccgaa gccgcttgga ataaggccgg      60
   tgtgcgtttg tctatatgtt attttccacc atattgccgt cttttggcaa tgtgagggcc     120
   cggaaacctg gccctgtctt cttgacgagc attcctaggg gtctttcccc tctcgccaaa     180
   ggaatgcaag gtctgttgaa tgtcgtgaag gaagcagttc ctctggaagc ttcttgaaga     240
   caaacaacgt ctgtagcgac cctttgcagg cagcggaacc ccccacctgg cgacaggtgc     300
   ctctgcggcc aaaagccacg tgtataagat acacctgcaa aggcggcaca accccagtgc     360
   cacgttgtga gttggatagt tgtggaaaga gtcaaatggc tctcctcaag cgtattcaac     420
   aaggggctga aggatgccca gaaggtaccc cattgtatgg gatctgatct ggggcctcgg     480
   tgcacatgct ttacatgtgt ttagtcgagg ttaaaaaaac gtctaggccc cccgaaccac     540
   ggggacgtgg ttttcctttg aaaaacacga tgataa                               576
<210> 5
   <211> 624
   <212> DNA
   <213> Artificial
<220>
   <223> tetR
<400> 5
   atgtctagat tagataaaag taaagtgatt aacagcgcat tagagctgct taatgaggtc      60
   ggaatcgaag gtttaacaac ccgtaaactc gcccagaagc taggtgtaga gcagcctaca     120
   ttgtattggc atgtaaaaaa taagcgggct ttgctcgacg ccttagccat tgagatgtta     180
   gataggcacc atactcactt ttgcccttta gaaggggaaa gctggcaaga ttttttacgt     240
   aataacgcta aaagttttag atgtgcttta ctaagtcatc gcgatggagc aaaagtacat     300
   ttaggtacac ggcctacaga aaaacagtat gaaactctcg aaaatcaatt agccttttta     360
   tgccaacaag gtttttcact agagaatgca ttatatgcac tcagcgctgt ggggcatttt     420
   actttaggtt gcgtattgga agatcaagag catcaagtcg ctaaagaaga aagggaaaca     480
   cctactactg atagtatgcc gccattatta cgacaagcta tcgaattatt tgatcaccaa     540
   ggtgcagagc cagccttctt attcggcctt gaattgatca tatgcggatt agaaaaacaa     600
   cttaaatgtg aaagtgggtc ttaa                                            624
<210> 6
   <211> 207
   <212> PRT
   <213> Artificial
<220>
   <223> TetR
<400> 6
   Met Ser Arg Leu Asp Lys Ser Lys Val Ile Asn Ser Ala Leu Glu Leu
   1               5                   10                  15
   Leu Asn Glu Val Gly Ile Glu Gly Leu Thr Thr Arg Lys Leu Ala Gln
               20                  25                  30
   Lys Leu Gly Val Glu Gln Pro Thr Leu Tyr Trp His Val Lys Asn Lys
           35                  40                  45
   Arg Ala Leu Leu Asp Ala Leu Ala Ile Glu Met Leu Asp Arg His His
       50                  55                  60
   Thr His Phe Cys Pro Leu Glu Gly Glu Ser Trp Gln Asp Phe Leu Arg
   65                  70                  75                  80
   Asn Asn Ala Lys Ser Phe Arg Cys Ala Leu Leu Ser His Arg Asp Gly
                   85                  90                  95
   Ala Lys Val His Leu Gly Thr Arg Pro Thr Glu Lys Gln Tyr Glu Thr
               100                 105                 110
   Leu Glu Asn Gln Leu Ala Phe Leu Cys Gln Gln Gly Phe Ser Leu Glu
           115                 120                 125
   Asn Ala Leu Tyr Ala Leu Ser Ala Val Gly His Phe Thr Leu Gly Cys
       130                 135                 140
   Val Leu Glu Asp Gln Glu His Gln Val Ala Lys Glu Glu Arg Glu Thr
   145                 150                 155                 160
   Pro Thr Thr Asp Ser Met Pro Pro Leu Leu Arg Gln Ala Ile Glu Leu
                   165                 170                 175
   Phe Asp His Gln Gly Ala Glu Pro Ala Phe Leu Phe Gly Leu Glu Leu
               180                 185                 190
   Ile Ile Cys Gly Leu Glu Lys Gln Leu Lys Cys Glu Ser Gly Ser
           195                 200                 205
<210> 7
   <211> 1473
   <212> DNA
   <213> herpes simplex virus
<400> 7
   atggacctct tggtcgacga gctgtttgcc gacatgaacg cggacggcgc ttcgccaccg      60
   cccccccgcc cggccggggg tcccaaaaac accccggcgg cccccccgct gtacgcaacg     120
   gggcgcctga gccaggccca gctcatgccc tccccaccca tgcccgtccc ccccgccgcc     180
   ctctttaacc gtctcctcga cgacttgggc tttagcgcgg gccccgcgct atgtaccatg     240
   ctcgatacct ggaacgagga tctgttttcg gcgctaccga ccaacgccga cctgtaccgg     300
   gagtgtaaat tcctatcaac gctgcccagc gatgtggtgg aatgggggga cgcgtacgtc     360
   cccgaacgca cccaaatcga cattcgcgcc cacggcgacg tggccttccc tacgcttccg     420
   gccacccgcg acggcctcgg gctctactac gaagcgctct ctcgtttctt ccacgccgag     480
   ctacgggcgc gggaggagag ctatcgaacc gtgttggcca acttctgctc ggccctgtac     540
   cggtacctgc gcgccagcgt ccggcagctg caccgccagg cgcacatgcg cggacgcgat     600
   cgcgacctgg gagaaatgct gcgcgccacg atcgcggaca ggtactaccg agagaccgct     660
   cgtctggcgc gtgttttgtt tttgcatttg tatctatttt tgacccgcga gatcctatgg     720
   gccgcgtacg ccgagcagat gatgcggccc gacctgtttg actgcctctg ttgcgacctg     780
   gagagctggc gtcagttggc gggtctgttc cagcccttca tgttcgtcaa cggagcgctc     840
   accgtccggg gagtgccaat cgaggcccgc cggctgcggg agctaaacca cattcgcgag     900
   caccttaacc tcccgctggt gcgcagcgcg gctacggagg agccaggggc gccgttgacg     960
   acccctccca ccctgcatgg caaccaggcc cgcgcctctg ggtactttat ggtgttgatt    1020
   cgggcgaagt tggactcgta ttccagcttc acgacctcgc cctccgaggc ggtcatgcgg    1080
   gaacacgcgt acagccgcgc gcgtacgaaa aacaattacg ggtctaccat cgagggcctg    1140
   ctcgatctcc cggacgacga cgcccccgaa gaggcggggc tggcggctcc gcgcctgtcc    1200
   tttctccccg cgggacacac gcgcagactg tcgacggccc ccccgaccga tgtcagcctg    1260
   ggggacgagc tccacttaga cggcgaggac gtggcgatgg cgcatgccga cgcgctagac    1320
   gatttcgatc tggacatgtt gggggacggg gattccccgg ggccgggatt taccccccac    1380
   gactccgccc cctacggcgc tctggatatg gccgacttcg agtttgagca gatgtttacc    1440
   gatgcccttg gaattgacga gtacggtggg tag                                 1473
<210> 8
   <211> 490
   <212> PRT
   <213> herpes simplex virus
<400> 8
   Met Asp Leu Leu Val Asp Glu Leu Phe Ala Asp Met Asn Ala Asp Gly
   1               5                   10                  15
   Ala Ser Pro Pro Pro Pro Arg Pro Ala Gly Gly Pro Lys Asn Thr Pro
               20                  25                  30
   Ala Ala Pro Pro Leu Tyr Ala Thr Gly Arg Leu Ser Gln Ala Gln Leu
           35                  40                  45
   Met Pro Ser Pro Pro Met Pro Val Pro Pro Ala Ala Leu Phe Asn Arg
       50                  55                  60
   Leu Leu Asp Asp Leu Gly Phe Ser Ala Gly Pro Ala Leu Cys Thr Met
   65                  70                  75                  80
   Leu Asp Thr Trp Asn Glu Asp Leu Phe Ser Ala Leu Pro Thr Asn Ala
                   85                  90                  95
   Asp Leu Tyr Arg Glu Cys Lys Phe Leu Ser Thr Leu Pro Ser Asp Val
               100                 105                 110
   Val Glu Trp Gly Asp Ala Tyr Val Pro Glu Arg Thr Gln Ile Asp Ile
           115                 120                 125
   Arg Ala His Gly Asp Val Ala Phe Pro Thr Leu Pro Ala Thr Arg Asp
       130                 135                 140
   Gly Leu Gly Leu Tyr Tyr Glu Ala Leu Ser Arg Phe Phe His Ala Glu
   145                 150                 155                 160
   Leu Arg Ala Arg Glu Glu Ser Tyr Arg Thr Val Leu Ala Asn Phe Cys
                   165                 170                 175
   Ser Ala Leu Tyr Arg Tyr Leu Arg Ala Ser Val Arg Gln Leu His Arg
               180                 185                 190
   Gln Ala His Met Arg Gly Arg Asp Arg Asp Leu Gly Glu Met Leu Arg
           195                 200                 205
   Ala Thr Ile Ala Asp Arg Tyr Tyr Arg Glu Thr Ala Arg Leu Ala Arg
       210                 215                 220
   Val Leu Phe Leu His Leu Tyr Leu Phe Leu Thr Arg Glu Ile Leu Trp
   225                 230                 235                 240
   Ala Ala Tyr Ala Glu Gln Met Met Arg Pro Asp Leu Phe Asp Cys Leu
                   245                 250                 255
   Cys Cys Asp Leu Glu Ser Trp Arg Gln Leu Ala Gly Leu Phe Gln Pro
               260                 265                 270
   Phe Met Phe Val Asn Gly Ala Leu Thr Val Arg Gly Val Pro Ile Glu
           275                 280                 285
   Ala Arg Arg Leu Arg Glu Leu Asn His Ile Arg Glu His Leu Asn Leu
       290                 295                 300
   Pro Leu Val Arg Ser Ala Ala Thr Glu Glu Pro Gly Ala Pro Leu Thr
   305                 310                 315                 320
   Thr Pro Pro Thr Leu His Gly Asn Gln Ala Arg Ala Ser Gly Tyr Phe
                   325                 330                 335
   Met Val Leu Ile Arg Ala Lys Leu Asp Ser Tyr Ser Ser Phe Thr Thr
               340                 345                 350
   Ser Pro Ser Glu Ala Val Met Arg Glu His Ala Tyr Ser Arg Ala Arg
           355                 360                 365
   Thr Lys Asn Asn Tyr Gly Ser Thr Ile Glu Gly Leu Leu Asp Leu Pro
       370                 375                 380
   Asp Asp Asp Ala Pro Glu Glu Ala Gly Leu Ala Ala Pro Arg Leu Ser
   385                 390                 395                 400
   Phe Leu Pro Ala Gly His Thr Arg Arg Leu Ser Thr Ala Pro Pro Thr
                   405                 410                 415
   Asp Val Ser Leu Gly Asp Glu Leu His Leu Asp Gly Glu Asp Val Ala
               420                 425                 430
   Met Ala His Ala Asp Ala Leu Asp Asp Phe Asp Leu Asp Met Leu Gly
           435                 440                 445
   Asp Gly Asp Ser Pro Gly Pro Gly Phe Thr Pro His Asp Ser Ala Pro
       450                 455                 460
   Tyr Gly Ala Leu Asp Met Ala Asp Phe Glu Phe Glu Gln Met Phe Thr
   465                 470                 475                 480
   Asp Ala Leu Gly Ile Asp Glu Tyr Gly Gly
                   485                 490
<210> 9
   <211> 624
   <212> DNA
   <213> Artificial
<220>
   <223> rtetR
<400> 9
   atgtctagat tagataaaag taaagtgatt aacagcgcat tagagctgct taatgaggtc      60
   ggaatcgaag gtttaacaac ccgtaaactc gcccagaagc ttggtgtaga gcagcctaca     120
   ctgtattggc atgtaaaaaa taagcgggct ttgctcgacg ccttagccat tgagatgtta     180
   gataggcacc atactcactt ttgcccttta aaaggggaaa gctggcaaga ttttttacgc      240
   aataacgcta aaagttttag atgtgcttta ctaagtcatc gcaatggagc aaaagtacat     300
   tcagatacac ggcctacaga aaaacagtat gaaactctcg aaaatcaatt agccttttta     360
   tgccaacaag gtttttcact agagaacgcg ttatatgcac tcagcgctgt ggggcatttt     420
   actttaggtt gcgtattgga agatcaagag catcaagtcg ctaaagaaga aagggaaaca     480
   cctactactg atagtatgcc gccattatta cgacaagcta tcgaattatt tgatcaccaa     540
   ggtgcagagc cagccttctt attcggcctt gaattgatca tatgcggatt agaaaaacaa 600
   cttaaatgtg aaagtgggtc ttaa 624
<210> 10
   <211> 207
   <212> PRT
   <213> Artificial
<220>
   <223> rTetR
<400> 10
   Met Ser Arg Leu Asp Lys Ser Lys Val Ile Asn Ser Ala Leu Glu Leu
   1               5                   10                  15
   Leu Asn Glu Val Gly Ile Glu Gly Leu Thr Thr Arg Lys Leu Ala Gln
               20                  25                  30
   Lys Leu Gly Val Glu Gln Pro Thr Leu Tyr Trp His Val Lys Asn Lys
           35                  40                  45
   Arg Ala Leu Leu Asp Ala Leu Ala Ile Glu Met Leu Asp Arg His His
       50                  55                  60
   Thr His Phe Cys Pro Leu Lys Gly Glu Ser Trp Gln Asp Phe Leu Arg
   65                  70                  75                  80
   Asn Asn Ala Lys Ser Phe Arg Cys Ala Leu Leu Ser His Arg Asn Gly
                   85                  90                  95
   Ala Lys Val His Ser Asp Thr Arg Pro Thr Glu Lys Gln Tyr Glu Thr
               100                 105                 110
   Leu Glu Asn Gln Leu Ala Phe Leu Cys Gln Gln Gly Phe Ser Leu Glu
           115                 120                 125
   Asn Ala Leu Tyr Ala Leu Ser Ala Val Gly His Phe Thr Leu Gly Cys
       130                 135                 140
   Val Leu Glu Asp Gln Glu His Gln Val Ala Lys Glu Glu Arg Glu Thr
   145                 150                 155                 160
   Pro Thr Thr Asp Ser Met Pro Pro Leu Leu Arg Gln Ala Ile Glu Leu
                   165                 170                 175
   Phe Asp His Gln Gly Ala Glu Pro Ala Phe Leu Phe Gly Leu Glu Leu
               180                 185                 190
   Ile Ile Cys Gly Leu Glu Lys Gln Leu Lys Cys Glu Ser Gly Ser
           195                 200                 205
<210> 11
   <211> 1008
   <212> DNA
   <213> Artificial
<220>
   <223> sequence coding for tTA fusion protein
<400> 11
   atgtctagat tagataaaag taaagtgatt aacagcgcat tagagctgct taatgaggtc     60
   ggaatcgaag gtttaacaac ccgtaaactc gcccagaagc taggtgtaga gcagcctaca    120
   ttgtattggc atgtaaaaaa taagcgggct ttgctcgacg ccttagccat tgagatgtta    180
   gataggcacc atactcactt ttgcccttta gaaggggaaa gctggcaaga ttttttacgt     240
   aataacgcta aaagttttag atgtgcttta ctaagtcatc gcgatggagc aaaagtacat    300
   ttaggtacac ggcctacaga aaaacagtat gaaactctcg aaaatcaatt agccttttta    360
   tgccaacaag gtttttcact agagaatgca ttatatgcac tcagcgctgt ggggcatttt    420
   actttaggtt gcgtattgga agatcaagag catcaagtcg ctaaagaaga aagggaaaca    480
   cctactactg atagtatgcc gccattatta cgacaagcta tcgaattatt tgatcaccaa    540
   ggtgcagagc cagccttctt attcggcctt gaattgatca tatgcggatt agaaaaacaa    600
   cttaaatgtg aaagtgggtc cgcgtacagc cgcgcgcgta cgaaaaacaa ttacgggtct    660
   accatcgagg gcctgctcga tctcccggac gacgacgccc ccgaagaggc ggggctggcg    720
   gctccgcgcc tgtcctttct ccccgcggga cacacgcgca gactgtcgac ggcccccccg    780
   accgatgtca gcctggggga cgagctccac ttagacggcg aggacgtggc gatggcgcat    840
   gccgacgcgc tagacgattt cgatctggac atgttggggg acggggattc cccgggtccg    900
   ggatttaccc cccacgactc cgccccctac ggcgctctgg atatggccga cttcgagttt    960
   gagcagatgt ttaccgatgc ccttggaatt gacgagtacg gtgggtag                1008
<210> 12
   <211> 335
   <212> PRT
   <213> Artificial
<220>
   <223> tTA fusion protein
<400> 12
   Met Ser Arg Leu Asp Lys Ser Lys Val Ile Asn Ser Ala Leu Glu Leu
   1               5                   10                  15
   Leu Asn Glu Val Gly Ile Glu Gly Leu Thr Thr Arg Lys Leu Ala Gln
               20                  25                  30
   Lys Leu Gly Val Glu Gln Pro Thr Leu Tyr Trp His Val Lys Asn Lys
           35                  40                  45
   Arg Ala Leu Leu Asp Ala Leu Ala Ile Glu Met Leu Asp Arg His His
       50                  55                  60
   Thr His Phe Cys Pro Leu Glu Gly Glu Ser Trp Gln Asp Phe Leu Arg
   65                  70                  75                  80
   Asn Asn Ala Lys Ser Phe Arg Cys Ala Leu Leu Ser His Arg Asp Gly
                   85                  90                  95
   Ala Lys Val His Leu Gly Thr Arg Pro Thr Glu Lys Gln Tyr Glu Thr
               100                 105                 110
   Leu Glu Asn Gln Leu Ala Phe Leu Cys Gln Gln Gly Phe Ser Leu Glu
           115                 120                 125
   Asn Ala Leu Tyr Ala Leu Ser Ala Val Gly His Phe Thr Leu Gly Cys
       130                 135                 140
   Val Leu Glu Asp Gln Glu His Gln Val Ala Lys Glu Glu Arg Glu Thr
   145                 150                 155                 160
   Pro Thr Thr Asp Ser Met Pro Pro Leu Leu Arg Gln Ala Ile Glu Leu
                   165                 170                 175
   Phe Asp His Gln Gly Ala Glu Pro Ala Phe Leu Phe Gly Leu Glu Leu
               180                 185                 190
   Ile Ile Cys Gly Leu Glu Lys Gln Leu Lys Cys Glu Ser Gly Ser Ala
   195 200 205
   Tyr Ser Arg Ala Arg Thr Lys Asn Asn Tyr Gly Ser Thr Ile Glu Gly
   210 215 220
   Leu Leu Asp Leu Pro Asp Asp Asp Ala Pro Glu Glu Ala Gly Leu Ala
   225 230 235 240
   Ala Pro Arg Leu Ser Phe Leu Pro Ala Gly His Thr Arg Arg Leu Ser
   245 250 255
   Thr Ala Pro Pro Thr Asp Val Ser Leu Gly Asp Glu Leu His Leu Asp
   260 265 270
   Gly Glu Asp Val Ala Met Ala His Ala Asp Ala Leu Asp Asp Phe Asp
   275 280 285
   Leu Asp Met Leu Gly Asp Gly Asp Ser Pro Gly Pro Gly Phe Thr Pro
   290 295 300
   His Asp Ser Ala Pro Tyr Gly Ala Leu Asp Met Ala Asp Phe Glu Phe
   305 310 315 320
   Glu Gln Met Phe Thr Asp Ala Leu Gly Ile Asp Glu Tyr Gly Gly
   325 330 335
<210> 13
   <211> 1008
   <212> DNA
   <213> Artificial
<220>
   <223> sequence coding for rtTA fusion protein
<400> 13
   atgtctagat tagataaaag taaagtgatt aacagcgcat tagagctgct taatgaggtc      60
   ggaatcgaag gtttaacaac ccgtaaactc gcccagaagc ttggtgtaga gcagcctaca     120
   ctgtattggc atgtaaaaaa taagcgggct ttgctcgacg ccttagccat tgagatgtta     180
   gataggcacc atactcactt ttgcccttta aaaggggaaa gctggcaaga ttttttacgc      240
   aataacgcta aaagttttag atgtgcttta ctaagtcatc gcaatggagc aaaagtacat     300
   tcagatacac ggcctacaga aaaacagtat gaaactctcg aaaatcaatt agccttttta      360
   tgccaacaag gtttttcact agagaacgcg ttatatgcac tcagcgctgt ggggcatttt     420
   actttaggtt gcgtattgga agatcaagag catcaagtcg ctaaagaaga aagggaaaca     480
   cctactactg atagtatgcc gccattatta cgacaagcta tcgaattatt tgatcaccaa     540
   ggtgcagagc cagccttctt attcggcctt gaattgatca tatgcggatt agaaaaacaa     600
   cttaaatgtg aaagtgggtc cgcgtacagc cgcgcgcgta cgaaaaacaa ttacgggtct     660
   accatcgagg gcctgctcga tctcccggac gacgacgccc ccgaagaggc ggggctggcg     720
   gctccgcgcc tgtcctttct ccccgcggga cacacgcgca gactgtcgac ggcccccccg     780
   accgatgtca gcctggggga cgagctccac ttagacggcg aggacgtggc gatggcgcat     840
   gccgacgcgc tagacgattt cgatctggac atgttggggg acggggattc cccgggtccg     900
   ggatttaccc cccacgactc cgccccctac ggcgctctgg atatggccga cttcgagttt     960
   gagcagatgt ttaccgatgc ccttggaatt gacgagtacg gtgggtag                 1008
<210> 14
   <211> 335
   <212> PRT
   <213> Artificial
<220>
   <223> rtTA fusion protein
<400> 14
   Met Ser Arg Leu Asp Lys Ser Lys Val Ile Asn Ser Ala Leu Glu Leu
   1               5                   10                  15
   Leu Asn Glu Val Gly Ile Glu Gly Leu Thr Thr Arg Lys Leu Ala Gln
               20                  25                  30
   Lys Leu Gly Val Glu Gln Pro Thr Leu Tyr Trp His Val Lys Asn Lys
           35                  40                  45
   Arg Ala Leu Leu Asp Ala Leu Ala Ile Glu Met Leu Asp Arg His His
       50                  55                  60
   Thr His Phe Cys Pro Leu Lys Gly Glu Ser Trp Gln Asp Phe Leu Arg
   65                  70                  75                  80
   Asn Asn Ala Lys Ser Phe Arg Cys Ala Leu Leu Ser His Arg Asn Gly
                   85                  90                  95
   Ala Lys Val His Ser Asp Thr Arg Pro Thr Glu Lys Gln Tyr Glu Thr
               100                 105                 110
   Leu Glu Asn Gln Leu Ala Phe Leu Cys Gln Gln Gly Phe Ser Leu Glu
           115                 120                 125
   Asn Ala Leu Tyr Ala Leu Ser Ala Val Gly His Phe Thr Leu Gly Cys
       130                 135                 140
   Val Leu Glu Asp Gln Glu His Gln Val Ala Lys Glu Glu Arg Glu Thr
   145                 150                 155                 160
   Pro Thr Thr Asp Ser Met Pro Pro Leu Leu Arg Gln Ala Ile Glu Leu
                   165                 170                 175
   Phe Asp His Gln Gly Ala Glu Pro Ala Phe Leu Phe Gly Leu Glu Leu
               180                 185                 190
   Ile Ile Cys Gly Leu Glu Lys Gln Leu Lys Cys Glu Ser Gly Ser Ala
           195                 200                 205
   Tyr Ser Arg Ala Arg Thr Lys Asn Asn Tyr Gly Ser Thr Ile Glu Gly
       210                 215                 220
   Leu Leu Asp Leu Pro Asp Asp Asp Ala Pro Glu Glu Ala Gly Leu Ala
   225                 230                 235                 240
   Ala Pro Arg Leu Ser Phe Leu Pro Ala Gly His Thr Arg Arg Leu Ser
                   245                 250                 255
   Thr Ala Pro Pro Thr Asp Val Ser Leu Gly Asp Glu Leu His Leu Asp
               260                 265                 270
   Gly Glu Asp Val Ala Met Ala His Ala Asp Ala Leu Asp Asp Phe Asp
           275                 280                 285
   Leu Asp Met Leu Gly Asp Gly Asp Ser Pro Gly Pro Gly Phe Thr Pro
       290                 295                 300
   His Asp Ser Ala Pro Tyr Gly Ala Leu Asp Met Ala Asp Phe Glu Phe
   305 310 315 320
   Glu Gln Met Phe Thr Asp Ala Leu Gly Ile Asp Glu Tyr Gly Gly
   325 330 335

## Description

### Technical Field

The present invention relates to processes for producing tetracycline (hereinafter referred to as "Tc") inducible gene expressing cell lines and uses thereof.

### Background Art

Recently, a system in which the expression of a gene of interest in a cell is controlled artificially (switched ON or switched OFF) depending on the presence or absence of tetracycline (hereinafter referred to as "Tc") or an analog thereof (hereinafter referred to as "Tc inducible gene expression system") has been used widely as a method of studying the functions of gene products. Hereinafter, Tc and an analog thereof are collectively called a "Tc compound". Examples of such a gene expression system include a so-called "Tet-Off expression system" in which transcription of a gene of interest is induced in the absence of the Tc compound and is suppressed in the presence of the Tc compound, and a so-called "Tet-On expression system" in which the transcription of a gene of interest is induced in the presence of the Tc compound and is suppressed in the absence of the Tc compound. Since a cell that allows such a system to function can allow the gene of interest to express in the same manner as in the case of a parent strain when necessary and can suppress the expression of the gene of interest when necessary, it is possible to analyze the functions of the gene of interest and gene products depending on the change in, for example, phenotype.

The former Tet-Off expression system is a system that utilizes the property of a Tet repressor (hereinafter referred to as a "TetR"), i.e. the property that it is bound to a tet operator sequence in the absence of the Tc compound and is not bound to the tet operator sequence in the presence of the Tc compound. Usually, this system uses a tTA expression vector that expresses a transactivator (hereinafter referred to as a "TA") and a gene-of-interest expression vector that introduces a gene of interest. Usually, the TA in the tTA expression vector is a fusion protein (hereinafter referred to as a "tTA") of a TetR and a transcriptional activation domain of herpes simplex virus 16. Furthermore, the gene-of-interest expression vector has a promoter region containing a tet operator and a minimal promoter unit. When a host cell into which these vectors have been transfected is cultured, the fusion protein tTA that has been expressed can be bound to the tet operator in the promoter region in the case of the absence of the Tc compound. Accordingly, the transcription of the gene of interest is induced (switched-On). On the other hand, in the case of the presence of the Tc compound, the Tc compound is bound to an inducible protein tTA that has been expressed. The tTA in this complex cannot bind to the tet operator in the promoter region. Consequently, the transcription of the gene of interest is suppressed (switched-Off).

The latter Tet-On expression system is a system that utilizes the property of an rTet repressor (hereinafter referred to as an "rTetR"), i.e. the property that it is bound to a tet operator sequence in the presence of the Tc compound and is not bound to the tet operator sequence in the absence of the Tc compound. Generally, this system also uses a TA expression vector and a gene-of-interest expression vector. The TA in the TA expression vector is a fusion protein (hereinafter referred to as an "rtTA") of rTetR and a transcriptional activation domain of herpes simplex VP16. When a host cell with these vectors transfected thereinto is cultured, the Tc compound is bound to the inducible protein rtTA that has been expressed, in the case of the presence of the Tc compound. Since this complex is bound to the tet operator in the promoter region, the transcription of the gene of interest is induced (switched-On). On the other hand, a fusion protein rtTA that has been expressed cannot bind to the tet operator sequence independently. Accordingly, in the case of the absence of the Tc compound, the rtTA cannot bind to the tet operator sequence in the promoter region. Consequently, transcription of the gene of interest is suppressed (switched-Off). In the production of cell lines in which such gene expression systems function, generally, it is checked through screening using a selection marker such as a drug-resistance marker whether various vectors have been transfected into host cells JP 11(1999)-506901A.

However, the gene expression systems that are controlled depending on the presence/absence of the Tc compound as described above are said to be not suitable for use for all cells in the same manner. Actually, it has been considered as a problem that it is difficult to obtain cell lines in which the above-mentioned Tc inducible gene expression systems function, for example, an early embryonic cell, germ-line cell, primary cultured cell, hematopoietic cell, neural cell, etc. Particularly, recently, a Nalm-6 cell, a human pre-B cell, is gaining attention since it allows highly-efficient gene targeting to be carried out. However, with respect to this Nalm-6 cell, it also has been considered as a problem that it is difficult to obtain cell lines in which the Tc inducible gene expression systems function.

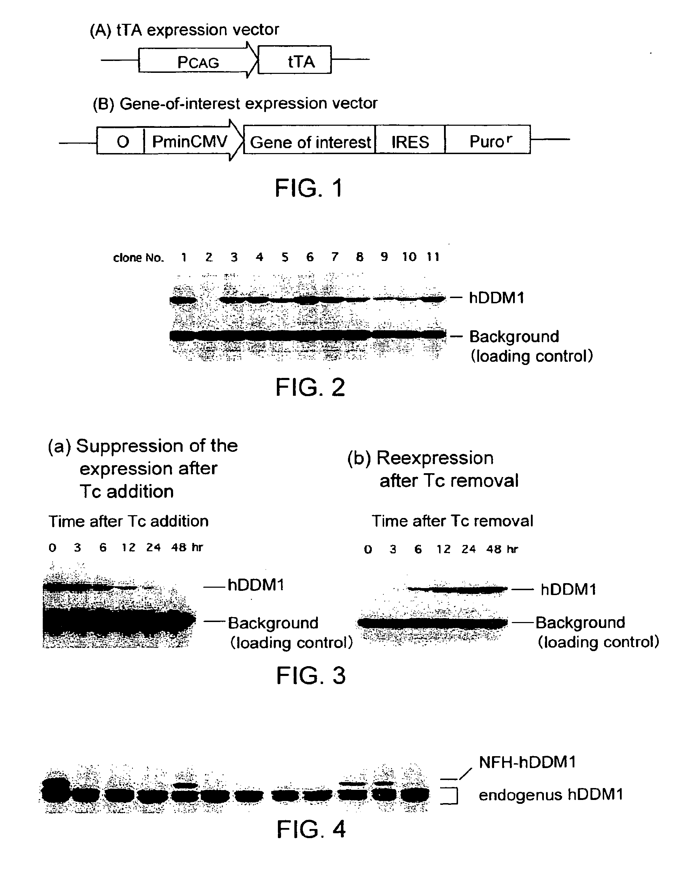

Yamazoe et al., DNA Repair 3, 1175-1185, 2004, is a review article relating to reverse genetic studies of the DNA damage response in the chicken B lymphocyte line DT40. Figure 2 shows strategies for producing cells conditionally null for essential genes. After heterozygous (+/-) mutant cells are generated, two strategies are described to make homozygous (-/-) mutant cells: (A) knock-in sequences of conditional inactivation into the remaining + allele, and (B) random integration of a transgene that expresses the deleted gene product. In (B), the target gene on the rescue plasmid is regulated by a Tet promoter and its expression can be monitored by a luciferase marker preceded by an IRES.

Shaikh and Nicholson, J. Biomol. Tech. 17(4) 282-292, 2006, reports a two-plasmid Tet-On system utilising a regulatory plasmid and a response plasmid. For the regulatory plasmid, rtTA (reverse tetracycline transactivator) was placed under either the CMV promoter or the cell-specific promoter neuronal specific enolase. For the response plasmid, the gene RAGE was cloned in pIRES-EGFP plasmid (Clontech) and the CMV promoter replaced with TREtight (modified seven copies of Tet-operon fused with CMVm promoter)

### Disclosure of Invention

In order to solve the problem that cell lines in which the Tc inducible gene expression systems function (hereinafter referred to as "inducible gene expression cell lines") cannot be obtained depending on the type of the cells, the present inventors focused on the method of screening cell lines in the case where the TA expression vector and gene-of-interest expression vector have been transfected into a host cell, as described below.

In the conventional method, after a TA expression vector is transfected into a host cell first, cell lines with the TA expression vectors transfected thereinto are selected using a selection marker with, for example, drug resistance of the vector. However, it was obvious that a TA expression vector had been transfected into each of a plurality of cell lines to be selected in this step, but it was found that considerably large variations in the expression activity (level of expression) of genes occurred among cell lines and cell clones when the expression was induced. This became clear through the assay described below that employed a control vector for checking the induced expression. That is, in the screening of the Tet-Off expression system, when control vectors (for instance, pTRE2-Luc (trade name), manufactured by Clontech Laboratories, Inc.) that induced expression of luciferase in the absence of the Tc compound were transfected into a plurality of cell lines into which TA expression vectors had been transfected, which were obtained in the same method as described above, the results showed that the levels of expression of luciferase were completely different from one another according to the respective cell lines. The fact that there is a problem in variations in the expression activity as described above means that it is not clear whether the expression of the gene of interest actually can be controlled when the expression is induced even if gene-of-interest expression vectors further are transfected into the selected cell lines. As described above, however, in the Tc inducible gene expression systems, it is necessary to allow a gene of interest to be expressed as in the case of a parent cell line by inducing expression when desired. Accordingly, the Tc inducible gene expression system is hardly functioning in the case of cell lines whose expression activity is too low when the expression is induced as compared to, for example, the parent strain. Furthermore, if the expression activity is too low, there is a possibility that when the expression is induced, the phenotype may differ from that of the parent cell line. Therefore, in conventional methods, it is not sufficient merely to select cell lines with the vectors transfected thereinto, using a selection marker of the TA expression vector. With respect to a plurality of cell lines obtained, the aforementioned assay that employs control vectors (luciferase expression vectors) is indispensable. Cell lines that exhibit sufficiently high level of luciferase expression are selected through the above-mentioned assay, and subsequently, the gene-of-interest expression vectors are transfected into the cell lines thus selected. However, even in the case of the cell lines with respect to which it was confirmed by the assay using the control vectors that luciferase was highly expressed, it is necessary not only that the gene-of-interest expression vectors are transfected but also that actually the expression of the gene of interest is controlled when the expression is induced. Furthermore, even if cell lines are obtained, each of which sufficiently expresses the gene of interest when the expression is induced, it does not mean that consequently desired cell lines in which the Tc inducible gene expression system functions with respect to the gene of interest are obtained unless the expression of the gene of interest is lost immediately when expression is not induced. Therefore, it is not sufficient only to select the cell lines with the vectors transfected thereinto, using a selection marker of the gene-of-interest expression vector. It is indispensable to check the presence or absence of the control of the expression of the gene of interest that is provided by the Tc compound with respect to the selected cell lines. As described above, in the conventional methods, in order to Tc inducible gene-of-interest expressing cell lines, after the respective vectors are transfected, not only the selection using the respective selection markers but also a further step of selection to be performed by an assay of the level of luciferase expression is indispensable. In addition, an assay of the expression level of the gene of interest also is indispensable. Therefore, for example, even in the case of cells, with respect to which constructions of Tc inducible gene expressing cell lines have been reported, the conventional methods involve a good deal of time and effort to obtain them.

Furthermore, as in the case of, for example, the aforementioned hematopoietic cells, a further longer period of time is required with respect to cells whose inducible gene-of-interest expressing cell lines actually have not been obtained. That is, with respect to such cells, it is necessary to perform the aforementioned assay of the level of luciferase expression or assay of the expression level of gene of interest endlessly with respect to a huge number of cell lines until the inducible gene-of-interest expressing cell lines are obtained. However, with respect to such cells, it is conceivable that the probability itself that the inducible gene-of-interest expressing cell lines are obtained is very low in the first place. Accordingly, even when the assay is performed with respect to a huge number of cell lines, the possibility that the gene-of-interest expressing cell lines are contained in those cell lines also is very low. In this manner, although the possibility that the desired cell lines are contained is very low, the presence or absence of the control of the expression of the gene of interest and the level of luciferase expression has to be checked with respect to a huge number of cell lines with the respective vectors transfected thereinto. Therefore, it takes a lot of time and thus the efficiency is very low. Furthermore, it also is conceivable that a low selection efficiency due to the necessity of the assay of, for example, the level of expression to be performed exhaustively with respect to a large amount of cell lines that have been confirmed to have the respective vectors transfected thereinto contributes to the fact that the gene-of-interest expressing cell lines have not been detected.

Therefore, the present inventors thought that, for example, an easy method capable of screening candidate cell lines likely to be the Tc inducible gene-of-interest expressing cell lines without checking the level of luciferase expression allowed the screening to be performed efficiently even in the case of, for example, a cell in which the probability itself of obtaining Tc inducible gene expressing cell lines was very low. That is, even when the assay with respect to the level of expression is not performed exhaustively as described above with respect to the cell lines with various vectors transfected thereinto, a method capable of screening candidate cell lines likely to be the desired cell lines makes it only necessary to check, for example, the control of the expression of the gene of interest in the presence or absence of the Tc compound in the end with respect to only the cell lines selected by the method. Accordingly, the inventors thought that Tc inducible gene-of-interest expression cell lines were screened with a very high efficiency, and consequently, Tc inducible gene-of-interest expression cell lines were able to be produced with a very high efficiency.

Therefore, the present invention is intended to provide a method of screening, easily with a high efficiency, so-called Tc inducible DNA expressing cell lines, in each of which the expression of DNA of interest can be regulated depending on the presence or absence of a Tc compound regardless of the type of cell, and a process for producing Tc inducible DNA expressing cell lines.

In order to achieve the above-mentioned object, the screening method of the present invention is a method of screening an inducible DNA expressing cell line that allows the expression of a DNA of interest to be regulated depending on the presence or absence of a Tc compound, wherein the Tc compound is Tc or a Tc analog, and the method includes the following steps (A) and (B):
(A) transfecting a transactivator expression vector and a DNA-of-interest expression vector into a host cell which is a Nalm-6 cell,
   where the transactivator expression vector includes:
   a polynucleotide sequence encoding a transactivator and a CAG promoter sequence that controls transcription of the polynucleotide sequence encoding a transactivator, with the polynucleotide sequence encoding a transactivator being arranged under control of the CAG promoter sequence,
   the transactivator is a protein that is switched to be bound or unbound to a tet operator sequence depending on the presence or absence of the Tc compound, and the transactivator is a fusion protein containing a Tet repressor and a transcriptional activation domain or a fusion protein containing a reverse Tet repressor and a transcriptional activation domain, and
   the DNA-of-interest expression vector includes:
      a tet operator sequence, a DNA-of-interest sequence, a promoter sequence that controls transcription of the DNA-of-interest sequence, a bicistronic regulatory sequence, and a polynucleotide sequence encoding a selection marker, with the bicistronic regulatory sequence being arranged between the DNA-of-interest sequence and the polynucleotide sequence encoding a selection marker under control of the tet operator sequence and the promoter sequence, and
(B) selecting cell lines, in each of which the selection marker in the DNA-of-interest expression vector has been expressed, from the host cell that has been subjected to transfection of the transactivator expression vector and the DNA-of-interest expression vector.

The production process of the present invention is a process for producing an inducible DNA expressing cell line that allows the expression of a DNA of interest to be regulated depending on the presence or absence of a Tc compound, wherein the inducible DNA expressing cell line is obtained by a method of screening an inducible DNA expressing cell line according to the present invention.

A production process of the present invention is a process for producing an inducible DNA knockout cell line that allows the expression of a DNA of interest to be regulated depending on the presence or absence of a Tc compound, wherein the Tc compound is Tc or a Tc analog, and the process includes the following steps (a) to (c):
(a) before or after the following step (b), knocking out one allele of an endogenous DNA of interest in a host cell which is a Nalm-6 cell,
(b) producing an inducible DNA expressing cell line by a process for producing an inducible DNA expressing cell line of the present invention,
   where a DNA-of-interest expression vector is a vector that includes, as a DNA-of-interest sequence, an exogenous DNA sequence with the same function as that of the endogenous DNA-of-interest sequence of the host cell, and
(c) knocking out the other allele of the endogenous DNA-of-interest sequence in the cell line obtained in step (b).

A production process of the present invention is a process for producing an altered DNA harboring knockout cell line in which an endogenous DNA has been knocked out and into which an altered DNA whose expression can be regulated depending on the presence or absence of a Tc compound has been transfected, wherein the Tc compound is Tc or a Tc analog, and the process includes the following steps (1) to (n):
(l) before or after the following step (m), knocking out one allele of an endogenous DNA-of-interest sequence in a host cell which is a Nalm-6 cell,
(m) producing an inducible DNA expressing cell line by a process for producing an inducible DNA expressing cell line of the present invention,
   where a DNA-of-interest expression vector is a vector that includes, as a DNA-of-interest sequence, an altered sequence of the endogenous DNA-of-interest sequence of the host cell, and
(n) knocking out the other allele of the endogenous DNA-of-interest sequence in the cell line obtained in step (m).

In the present invention, the "DNA of interest" and "DNA-of-interest sequence" are not particularly limited. Examples thereof include a gene, a partial DNA sequence of a gene, a DNA sequence including a gene, and a DNA sequence whose function is unknown. In the present invention, hereinafter, the inducible DNA expressing cell line that allows the expression of a DNA of interest to be regulated depending on the presence or absence of a Tc compound is referred to as an "inducible DNA expressing cell line" and such a function also is referred to as "Tc inducibility" or "Tc responsiveness". The aforementioned "inducible DNA expressing cell line" embraces a form in which a DNA of interest is expressed in the absence of the Tc compound and the expression of the DNA of interest is lost in the presence of the Tc compound (Tet-Off expression system) and a form in which the DNA of interest is expressed in the presence of the Tc compound and the expression of the DNA of interest is lost in the absence of the Tc compound (Tet-On expression system). In the Tet-Off expression system, induction of the expression denotes a state in the absence of the Tc compound, while noninduction of the expression denotes a state in the presence of the Tc compound. On the other hand, in the Tet-On expression system, induction of the expression denotes a state in the presence of the Tc compound, while noninduction denotes a state in the absence of the Tc compound. Whether the expression of the DNA of interest is induced in the absence or presence of the Tc compound depends on, for example, the type of transactivator (a tTA fusion protein or rtTA fusion protein). In the present invention, loss of expression also can be called suppression of expression. Hereinafter, in the present invention, an "inducible DNA expressing cell line" also is referred to as an "inducible gene expressing cell line", and a "DNA-of-interest expression vector" as a "gene-of-interest expression vector".

Moreover, a fusion protein containing TetR and a transcriptional activation domain is referred to as a "tTA fusion protein", a polynucleotide sequence encoding it as a "tTA coding sequence", a fusion protein containing rTetR and a transcriptional activation domain as an "rtTA fusion protein", and a polynucleotide sequence encoding it as an "rtTA coding sequence". A polynucleotide sequence encoding a TA is referred to as a "TA coding sequence", and a polynucleotide sequence encoding a selection marker as a "selection marker coding sequence".

According to the present invention, the use of the TA expression vector and DNA-of-interest expression vector with the aforementioned structures makes it possible to select cell lines with a very high probability of being inducible gene-of-interest expressing cell lines in step (B) described above. In the case of screening of a Tet-Off system, the cell lines selected in step (B) have a very high probability of being cell lines that exhibit the behavior of expressing the DNA of interest in the absence of the Tc compound and suppressing the expression of the DNA of interest in the presence of the Tc compound. On the other hand, in the case of screening of a Tet-On system, the cell lines selected in step (B) have a very high probability of being cell lines that exhibit the behavior of expressing the DNA of interest in the presence of the Tc compound and suppressing the expression of the DNA of interest in the absence of the Tc compound.

In the case of conventional methods, as described above, even when transfection of the TA expression vector and gene-of-interest expression vector was observed, it didn't indicate the possibility that the cell lines were inducible gene expressing cell lines. Accordingly, there were no other methods than that in which a huge number of cell lines with vectors transfected simply thereinto were assayed one by one to be checked whether they are inducible gene-of-interest expressing cell lines. Even when all the cell lines were assayed, as a result, for example, the gene of interest was not expressed sufficiently when expression was induced or the gene of interest was expressed even when expression was not induced, i.e. there was a very high probability that no inducible gene-of-interest expressing cell lines existed. Accordingly, there was a problem in that no good results were produced although it took a lot of time and effort. However, in the present invention, using the gene-of-interest expression vector (DNA-of-interest expression vector) as described above, the following is possible by merely checking the expression of a selection marker of the gene-of-interest expression vector when the expression is induced. First, cell lines, into which both the TA expression vector and the gene-of-interest expression vector have been transfected, can be selected by mere checking of the expression of the selection marker. This is because of the following reasons: If the TA expression vector has been transfected, the TA is expressed inside the host cell, and therefore when expression is induced (for example, in the absence of the Tc compound in the case of the Tet-Off system and in the presence of the Tc compound in the case of the Tet-On system), the expression TA is bound to the tet operator of the gene-of-interest expression vector to induce transcription of a DNA sequence located downstream of the operator. Accordingly, the expression of the selection marker of the gene-of-interest expression vector denotes that the cell lines are those also with the TA expression vector transfected thereinto. Second, it can be proved by mere checking of the expression of the selection marker that the cell lines are those in which a certain level of expression of the gene of interest is obtained, for example, in the absence (Tet-Off) or presence (Tet-On) of the Tc compound. This is because the gene of interest and the selection marker are arranged, with a bicistronic regulatory sequence being interposed therebetween, in the gene-of-interest expression vector. That is, the fact that the selection marker of the gene-of-interest expression vector is expressed when expression is induced denotes that the gene of interest (DNA of interest) under the control of the bicistronic regulatory sequence also is expressed. Therefore, it can be proved by mere checking of the expression of the selection marker that a certain level of expression of the gene of interest is obtained. Third, it also is possible to surmise the degree of expression of the gene of interest from the level of expression of the selection marker. In conventional methods, as described earlier, in order to select cell lines whose level of expression is sufficiently high when expression is induced, luciferase assay using a control vector is required with respect to cell lines with the TA expression vector transfected thereinto. However, according to the present invention, since the level of expression of the selection marker can be surmised indirectly as the level of expression of the gene of interest, such assay is no longer required. Accordingly, in the present invention, checking of transfection through the expression of the selection marker makes it possible simultaneously to screen cell lines, into which both the vectors have not been transfected, and cell lines whose level of expression is low when expression is induced. Therefore, candidate cell lines with very high probability of being the inducible gene-of-interest expressing cell lines (inducible DNA expressing cell lines) can be screened. Moreover, since the cell lines selected in step (B) are candidate cell lines highly likely to be the inducible gene-of-interest expressing cell lines that exhibit Tc responsiveness, for example, it is sufficient to check the control of expression of the gene of interest that is achieved by the Tc compound with respect to only the candidate cell lines. Accordingly, as compared to conventional methods, the whole method of screening Tc inducible gene expressing cell lines can be performed easily. As described above, since the present invention allows screening to be performed easily and efficiently, it is possible to obtain Tc inducible gene expressing cell lines that can maintain a required level of expression of a gene of interest artificially in a shorter time with higher efficiency as compared to conventional cases and that can lose the expression at a desired time. Furthermore, similarly with respect to, for example, hematopoietic cells from which it has been stochastically difficult to obtain Tc inducible gene expressing cell lines, it can be said that the possibility that the desired cell lines can be obtained has been improved. Thus, the present invention makes it possible to, for example, analyze the functions of gene products in cells that have not been studied sufficiently until now, and therefore the technique of the present invention can be said to be very useful in the developments of drugs and clinical diagnosis kits and the fields of life science and clinical medicine.

### Brief Description of Drawings

FIG. 1 shows diagrams, each of which illustrates a partial structure of a vector used in Example 1 of the present invention; FIG. 1(A) shows a diagram illustrating a partial structure of a TA expression vector and FIG. 1(B) shows a diagram illustrating a partial structure of a gene-of-interest expression vector.
FIG. 2 is a photograph showing the expression of an NFH-hDDM1 protein in each puromycin-resistant cell line, into which a gene-of-interest expression vector (pTRE-tight-NFH-hDDM1-IRES-Puro vector) obtained through cloning of an hDDM1 gene has been transfected, in Example 1.
FIG. 3(a) is a photograph showing the expression of an NFH-hDDM1 protein in a puromycin-resistant cell line, into which an NFH-HDDM1 gene with Tc added thereto has been transfected, in Example 1, and FIG. 3(b) is a photograph showing the expression of an NFH-hDDM1 protein in the puromycin-resistant cell line, after Tc has been removed, in Example 1.
FIG. 4 is a photograph showing the expression of an NFH-hDDM1 protein in neomycin-resistant cell lines, into each of which an NFH-hDDM1 gene has been transfected, in the presence of Tc in Example 2.
FIG. 5 shows photographs, each of which shows the expression of an NFH-hDDM1 protein in a neomycin-resistant cell line, into which an NFH-hDDM1 gene has been transfected, in Example 2; FIG. 5(a) shows the result after Tc was removed, and FIG. 5(b) the result after the Tc was added thereto again.
FIG. 6 is a schematic view showing the outline of the process for producing a Tc-inducible knockout cell line in Example 3.
FIG. 7(A) is a photograph of an autoradiography, with which the genotype of an endogenous hDDM1 gene locus was checked in Example 3, and FIG. 7(B) is a photograph showing the expression of an hDDM1 protein.
FIG. 8 is an autoradiograph showing a cleavage pattern of a purified genome of the Tc-inducible knockout cell line in Example 3.
FIG. 9(A) is an autoradiograph, with which the genotype of an endogenous hDDM1 gene locus was checked in Example 4, and FIG. 9(B) is a photograph showing the expressions of an endogenous hDDM1 protein and an NFH-hDDM1 protein.
FIG. 10 is an autoradiograph showing a cleavage pattern of a purified genome of an altered gene knockout cell line in Example 4.
FIG. 11 is a photograph showing the expression of an NFH-hDDM1 protein in each altered gene knockout cell line in Example 5.

### Best Mode for Carrying Out the Invention

### <Screening Method>

As described above, the screening method of the present invention can be used for screening of Tc inducible DNA expressing cell lines with respect to a host cell which is a Nalm-6 cell. In the case of Nalm-6 cell, it was difficult to obtain the constitutive high expression of a gene of interest in the Tet-Off expression system (or the constitutive high expression of a gene of interest in the Tet-On expression system) in conventional methods. However, according to the present invention, Tc inducible gene expressing cell lines can be obtained, each of which constitutively express a gene of interest when the expression is induced and suppresses the expression of the gene of interest when the expression is not induced. The present invention is not limited thereto.

### TA Expression Vector

The TA expression vector of the present invention is a vector that expresses a TA. As described above, it includes the TA coding sequence and a CAG promoter sequence that controls transcription of the TA coding sequence, and the TA coding sequence is arranged under the control of the promoter sequence.

The aforementioned TA is a protein that is switched to be bound or unbound to a tet operator sequence depending on the presence or absence of the Tc compound. The TA is a fusion protein (tTA fusion protein) containing a Tet repressor and a transcriptional activation domain or a fusion protein (rtTA fusion protein) containing a reverse Tet repressor and a transcriptional activation domain. With respect to the TA expression vectors of the present invention, a vector in which the TA is the tTA fusion protein is referred to as a "tTA expression vector" and a vector in which the TA is the rtTA fusion protein as an "rtTA expression vector". Hereinafter, in the present invention, "functionally arranged" and "functionally bound" denote that the object is arranged or bound in the state where intended functions thereof can be exhibited.

The promoter is a CAG promoter. The CAG promoter is known as a synthetic promoter containing a cytomegalovirus enhancer sequence, a chicken beta-actin promoter sequence, and a rabbit beta-globin exon sequence. For example, the CAG promoter may be prepared by PCR or cloning based on the aforementioned each sequence thereof or also can be prepared from a commercial vector or a plasmid pCAGGS (Niwa et al., 1991, Gene, 108:193-200) including a CAG promoter. In the present invention, the promoter sequence is functionally arranged so as to control transcription of the TA coding sequence.

Preferably, the TA expression vector includes, for example, a polyadenylation signal sequence in addition to the aforementioned sequences. Preferably, the polyadenylation signal sequence is functionally arranged, for example, on the downstream region (3' end region) of the TA coding sequence. Generally, the polyadenylation signal sequence is a signal for adding an adenine nucleotide chain (poly A sequence) consisting of 50 to 250 nucleotides to the 3' end of mRNA of a eukaryotic cell, and the poly A sequence is added by a poly A polymerase immediately after transcription and exhibits the function of protecting mRNA from degrading. The type of the polyadenylation signal sequence is not particularly limited, and examples thereof include an SV40 polyadenylation signal sequence and a beta-globin polyadenylation signal sequence.

The fusion protein (tTA fusion protein) containing TetR and a transcriptional activation domain is bound to a tet operator in the absence of the Tc compound and thereby activates transcription of a DNA of interest. Therefore, the tTA expression vector is useful as a tool for the so-called "Tet-Off expression system". On the other hand, the fusion protein (rtTA fusion protein) containing rTetR and a transcriptional activation domain is bound to a tet operator in the presence of the Tc compound and thereby activates transcription of a DNA of interest. Therefore, the rtTA expression vector is useful as a tool for the so-called "Tet-On expression system".

The tTA fusion protein is a fusion protein containing TetR and a transcriptional activation domain. The TetR of the tTA fusion protein is not particularly limited. A conventionally known example thereof is a wild-type TetR, and the class thereof (for instance, A, B, C, D, or E) is not particularly limited. Particularly, a wild-type TetR derived from Tn10 (Gossen, M et al., Proc. Natl. Acad. Sci. USA. 1992; 89:5547-5551) is preferable. The polynucleotide sequence encoding TetR (hereinafter referred to as a "TetR coding sequence") can be prepared by, for example, PCR or cloning based on a TetR amino acid sequence or a TetR coding sequence. Examples of the TetR coding sequence and the TetR amino acid sequence are indicated in SEQ ID NOs: 5 and 6, respectively, but the present invention is not limited thereto.

The transcriptional activation domain in the tTA fusion protein is a polypeptide that directly or indirectly activates transcription. It is not particularly limited as long as it is functionally bound to the TetR to form a fusion protein when being expressed. Therefore, the tTA coding sequence is preferably a chimera DNA that encodes a fusion protein and that includes the polynucleotide encoding the transcriptional activation domain and the TetR coding sequence that are bound in-frame.

Examples of the transcriptional activation domain include those known conventionally such as an acidic activation domain, proline-rich transcriptional activation domain, serine-threonine-rich transcriptional activation domain, and glutamine-rich activation domain. Examples of the acidic activation domain include herpes simplex virus virion protein 16 (hereinafter referred to as "VP16"). The amino acid sequence of VP16 is disclosed in, for example, Labow et al. ((1990) Mol. Cell Biol. 10:3343-3356) and Baim et al. ((1991) Proc. Natl. Acad. Sci. USA 88:5072-5076). Preferably, it contains at least an activation domain, specifically, a domain between positions 364 - 490 (127 amino acid residues) on the C-terminal region among all the amino acid sequences of VP16. The nucleotide sequence encoding VP16 (hereinafter referred to as a "VP16 coding sequence") may be prepared by, for example, PCR or cloning based on the amino acid sequence and the nucleotide sequence of VP16 or also can be prepared from a commercial vector containing the VP16 coding sequence. Examples of the VP16 coding sequence and the amino acid sequence of the VP16 are indicated in SEQ ID NOs: 7 and 8, respectively, but the present invention is not limited thereto.

Besides these, examples of the transcriptional activation domain also include a leucine zipper domain, helix-loop-helix domain, and zinc finger domain.

The rtTA fusion protein is a fusion protein of reverse TetR (rTetR) and a transcriptional activation domain. The rTetR is not particularly limited as long as it is polypeptide whose binding property has been changed due to a mutation of a wild-type TetR, i.e. polypeptide that is bound to a tet operator in the presence of the Tc compound and that is not bound to the tet operator in the absence of the Tc compound. Specific examples include a polypeptide in which at least one amino acid in an amino acid sequence of a wild-type TetR has been mutated, more specifically, a polypeptide in which at least one amino acid, preferably four amino acids among those at positions 71, 95, 101 and 102 in the amino acid sequence of a wild-type TetR (Tn10-derived wild-type TetR) have been mutated. The polynucleotide sequence encoding rTetR (hereinafter referred to as an "rTetR coding sequence") can be prepared by, for example, PCR or cloning based on the amino acid sequence of the rTetR or the rTetR coding sequence. The amino acid sequence of the rTetR or the rTetR coding sequence has been reported in, for example, Hillen and Berens (Annual. Rev. Microbiol. 1994:48 345-369) and Gossen et al. (Science 1995:268 1766-1769). Examples of the rTetR coding sequence and the amino acid sequence of the rTetR are indicated in SEQ ID NOs: 9 and 10, respectively, but the present invention is not limited thereto.

The transcriptional activation domain in the rtTA fusion protein also is a polypeptide that directly or indirectly activates transcription as in the case of the aforementioned tTA fusion protein. It is not particularly limited as long as it is functionally bound to the rTetR to form a fusion protein when being expressed. Accordingly, the rtTA coding sequence is preferably a chimera DNA that encodes a fusion protein and that includes the polynucleotide encoding the transcriptional activation domain and the rTetR coding sequence that are bound in-frame. The rtTA fusion protein coding sequence may be prepared by, for example, PCR or cloning based on the amino acid sequence of the rTetR or rTetR coding sequence, or also can be prepared from a commercial vector (for instance, a pTet-On vector (trade name), manufactured by Clontech Laboratories, Inc.).

An example of the tTA coding sequence is indicated in SEQ ID NO: 11, that of the amino acid sequence of the tTA fusion protein in SEQ ID NO: 12, that of the rtTA coding sequence in SEQ ID NO: 13, and that of the amino acid sequence of the rtTA fusion protein in SEQ ID NO: 14, respectively, but the present invention is not limited to these sequences.

In the present invention, the Tc compound may be either Tc or a Tc analog. Examples of the Tc analog include anhydrotetracycline, doxycycline, and cyanotetracycline. Even in the case of compounds that are not classified as a Tc analog, they are included in the Tc compounds as long as they exhibit similar functions as those of Tc and a Tc analog of the present invention. Furthermore, any one of the Tc compounds may be used or two or more of them may be used in combination.

The TA expression vector further may contain a selection marker coding sequence. The type of the selection marker is not particularly limited and examples thereof include those described later.

Specific examples of the TA expression vector of the present invention include a structure containing a CAG promoter sequence and a TA coding sequence that are arranged in this order from the upstream region and a structure containing a promoter sequence, a TA coding sequence, and a drug-resistance marker coding sequence that are arranged in this order from the upstream region.

### DNA-of-interest Expression Vector

The DNA-of-interest expression vector of the present invention is a vector for introducing a DNA of interest into the genome of a host cell by transfection. As described above, it includes a tet operator sequence, a DNA-of-interest sequence, a promoter sequence for controlling transcription of the DNA-of-interest sequence, a bicistronic regulatory sequence, and a selection marker coding sequence. The bicistronic regulatory sequence is arranged between the DNA-of-interest sequence and the selection marker coding sequence under the control of the tet operator sequence and the promoter sequence.

In the DNA-of-interest expression vector of the present invention, the type of the promoter is not particularly limited and can be determined suitably according to, for example, the biological species of the host cell that is employed in the screening method of the present invention. Examples of the promoter sequence include a minimum promoter sequence that initiates transcription of a DNA of interest. Generally, the term "minimum promoter sequence" denotes a partial promoter sequence of a domain that determines the transcription initiation site but cannot initiate transcription efficiently by itself. The activity of such a minimum promoter sequence depends on binding of TA to the tet operator sequence that is controlled by the Tc compound. Specifically, when the TA is a tTA fusion protein, the tTA fusion protein is bound to the tet operator sequence to be activated in the absence of the Tc compound, and when the TA is an rtTA fusion protein, the rtTA fusion protein is bound to the tet operator sequence to be activated in the presence of the Tc compound.

The minimum promoter sequence is not particularly limited. Specific examples thereof include a human cytomegalovirus (CMV)-derived promoter, and preferably a polynucleotide region between +75 to -53 or +75 to -31 can be used. The minimum CMV promoter may be prepared by, for example, PCR or cloning based on the sequence thereof or also can be prepared from a commercial vector (for instance, pC1-neo (trade name), manufactured by Promega) containing a minimum CMV promoter. An example of the sequence of the minimum CMV promoter is indicated in SEQ ID NO: 1, but the present invention is not limited thereto. Besides this, a conventionally known minimum promoter sequence can be used, and, for example, a minimum promoter derived from a promoter that controls transcription of a gene encoding thymidine kinase (tk) also can be used. A specific example is a herpes simplex virus-derived tk minimum promoter (Mc Knight et al. (1984) Cell. 37:253-262).

Preferably, the promoter sequence is arranged functionally (adjoined) with respect to the tet operator sequence. Generally, it is arranged on the downstream region (i.e. on the 3' region) of the tet operator sequence. Specifically, it is preferable that the promoter sequence and the tet operator sequence be functionally arranged (bound) at a suitable interval for initiation of transcription of the promoter (as well as, for example, a DNA of interest located downstream of the promoter) through, for example, binding of a TA (for instance, a tTA fusion protein or an rtTA fusion protein) to the tet operator sequence. The interval between the promoter sequence and the tet operator sequence is not particularly limited, and the promoter sequence can be arranged, for example, about 200 to 400 base pairs downstream of the tet operator sequence. In the case of screening of the Tet-Off system, the tet operator sequence is, for example, a sequence that is bound to a Tet repressor in the transactivator in the absence of the Tc compound and that is not bound to the Tet repressor in the transactivator in the presence of the Tc compound. Furthermore, in the case of screening of the Tet-On system, the tet operator sequence is, for example, a sequence that is bound to a reverse Tet repressor in the transactivator in the presence of the Tc compound and that is not bound to the reverse Tet repressor in the transactivator in the absence of the Tc compound.

In the DNA-of-interest expression vector of the present invention, one tet operator sequence may be arranged but it is preferable that a plurality of tet operator sequences be arranged successively. The copy number thereof (the number of successive sequences) is, for example, at least 2, preferably 2 to 10, and more preferably 7. The "tet operator sequence" is not particularly limited and may be a tet operator of any class (for instance, A, B, C, D, or E), and different tet operator sequences may be arranged successively. The phrase "arranged successively" denotes that the tet operator sequences may be arranged directly successively or may be arranged indirectly successively, for example, with a few bases being interposed therebetween. The tet operator sequence and a sequence including tet operator sequences arranged successively may be prepared by, for example, PCR or cloning based on the polynucleotide sequence of the tet operator or also can be prepared from a commercial vector (for instance, a pTRE-tight vector (trade name), manufactured by Clontech Laboratories, Inc.) containing the tet operator sequence. Examples of the tet operator sequence and the sequence containing a plurality of tet operator sequences are indicated in SEQ ID NOs: 2 and 3, respectively, but they are not limited thereto.

The "bicistronic regulatory sequence" denotes a regulatory sequence that provides the bicistronic nature, and the "bicistronic nature" denotes that two functionable proteins are expressed from one mRNA. Examples of the bicistronic regulatory sequence include the internal ribosomal entry site (IRES) sequence.

The bicistronic regulatory sequence is preferably the IRES sequence. In the DNA-of-interest expression vector of the present invention, it is preferable that the bicistronic regulatory sequence (for instance, the IRES sequence) be arranged between the DNA-of-interest sequence and the selection marker coding sequence, for example, downstream of a promoter sequence. The positional relationship between the DNA-of-interest sequence and the selection marker coding sequence is not particularly limited. For example, the DNA-of-interest sequence and the selection marker coding sequence may be arranged functionally on the upstream region and the downstream region, respectively, or the selection marker coding sequence and the DNA-of-interest sequence may be arranged functionally on the upstream region and the downstream region, respectively, with the IRES sequence being interposed therebetween. Particularly, it is preferable that the DNA-of-interest sequence, the IRES sequence, and the selection marker coding sequence be arranged from the upstream region in this order. This order allows, for example, the selection marker to be subjected to transcription subsequent to the transcription of the DNA-of-interest sequence. Accordingly, when, for example, the expression of the selection marker is observed, it can be judged with very high reliability that the DNA-of-interest sequence also is expressed. The IRES sequence is not particularly limited but is preferably, for example, the encephalomyocarditis virus (ECMV)-derived IRES sequence. The IRES sequence is described in, for example, US 4,937,190, JP 2002-514086A, and JP 2001-500021A. The IRES sequence may be synthesized by, for example, PCR or cloning based on the polynucleotide sequence of the IRES or also can be prepared from a commercial vector containing the IRES sequence. An example of the encephalomyocarditis virus-derived IRES sequence is indicated in SEQ ID NO: 4, but it is not limited thereto.

As described above, when the selection marker coding sequence is arranged on the downstream region of the IRES sequence, for example, a polyadenylation signal sequence further may be bound to the downstream region (3' end region) of the selection marker coding sequence. The type of the polyadenylation signal sequence is not particularly limited. Examples thereof include an SV40 polyadenylation signal sequence and a beta-globin polyadenylation signal sequence.

The selection marker coding sequence is not particularly limited, and examples thereof include sequences encoding markers such as a known drug-resistance marker, fluorescent protein marker, and cell surface receptor marker. The above-mentioned drug-resistance marker is not particularly limited and examples thereof include a neomycin-resistant marker, zeocin-resistant marker, puromycin-resistant marker, hygromycin-resistant marker, and histidinol-resistant marker. Examples of the above-mentioned fluorescent protein marker include a green fluorescent protein (GFP), mutated GFP (enhanced GFP, EGFP), and destabilized variant of EGFP (d2EGFP (2hr half-life: short half life). Furthermore, examples of the enzyme marker include luciferase and beta-galactosidase. These selection marker coding sequences may be synthesized by, for example, PCR according to the sequences thereof or also can be prepared from commercial vectors (for instance, pC1-neo (trade name), manufactured by Promega) containing the selection marker coding sequences. When the TA expression vector contains a selection marker, it is preferable that the selection marker of the DNA-of-interest expression vector is different from that of the TA expression vector.

In the DNA-of-interest expression vector of the present invention, the type of the DNA of interest is not limited by any means and can be set suitably according to the intended use. Examples of the DNA of interest include a gene, a partial sequence of a gene, a sequence including a gene, and a DNA sequence whose function is unknown. Specifically, as described later, for example, when it is intended to knock out an endogenous gene of a host cell, the DNA-of-interest sequence can be, for example, a sequence having the same sequence or the same function as that of the endogenous gene or a partial sequence thereof. Furthermore, as described later, when it is intended to transfect an altered gene in which, for example, a tag has been added or a base has been substituted, deleted, or added, the sequence of interest can be an altered DNA sequence in which, for example, a tag has been added to the endogenous gene or a partial sequence thereof or a base of the endogenous gene or a partial sequence thereof has been substituted, deleted, or added.

A specific example of the DNA-of-interest expression vector of the present invention is a structure including, for example, from the upstream region, a sequence containing tet operator sequences arranged a plurality of times (at least twice, or, for example, seven times) successively, a promoter sequence (for instance, a minimum CMV promoter sequence), a DNA-of-interest sequence, the IRES sequence, and a drug-resistance marker coding sequence that are arranged in this order. The DNA-of-interest expression vector of the present invention is not limited to this example.

Furthermore, in addition to the vectors containing a DNA-of interest sequence (hereinafter also referred to as "first DNA-of-interest expression vectors") as described above, examples of the DNA-of-interest expression vector of the present invention include the following second vectors. That is, a second DNA expression vector of the present invention includes a tet operator sequence, a cloning site that can bind a DNA-of-interest sequence, a promoter sequence that controls transcription of the DNA-of-interest sequence that is transfected into the cloning site, a bicistronic regulatory sequence, and a selection marker coding sequence. The bicistronic regulatory sequence is arranged between the cloning site and the selection marker coding sequence under the control of the tet operator sequence and the promoter sequence. This is the same as the first DNA-of-interest expression vector of the present invention except that the cloning site is included instead of the DNA-of-interest sequence.

For example, before the second DNA-of-interest expression vector of the present invention is used in the screening method of the present invention, a DNA-of-interest sequence may be ligated to the cloning site. The vector thus obtained is the aforementioned first DNA-of-interest expression vector.

In the second DNA-of-interest expression vector of the present invention, the cloning site is not particularly limited as long as it allows a DNA-of-interest sequence to be ligated thereto and as long as it is a site where, when the DNA-of-interest sequence is ligated thereto, transcription of the DNA of interest occurs under the control of the promoter sequence. The cloning site is preferably a so-called multicloning site (MCS) because it makes ligation easy regardless of the type of the terminal sequence of the DNA-of-interest sequence. The DNA of interest that is ligated to the cloning site can be determined arbitrarily and, for example, the type and origin thereof are not limited by any means.

As described earlier, the screening method of the present invention includes the following steps (A) and (B):
(A) transfecting a TA expression vector and a DNA-of-interest expression vector into a host cell which is a Nalm-6 cell, and
(B) selecting cell lines, in each of which the selection marker in the DNA-of-interest expression vector has been expressed, from the host cell that has been subjected to transfection of the TA expression vector and the DNA-of-interest expression vector.

In step (A), the TA expression vector and the DNA-of-interest expression vector may be transfected into the host cell separately (sequential transfection) in the same manner as in conventional cases, but as described later, a method of transfecting (cotransfecting) them simultaneously also is possible, which is different from conventional methods.

It is preferable that in step (B), cell lines be selected that express the selection marker when expression is induced. In the case of screening of the Tet-Off system using a tTA expression vector, the phrase "when the expression is induced" denotes, for example, a state in the absence of the Tc compound. Specifically, it is preferable that in step (B), cell lines that express the selection marker in the DNA-of-interest expression vector in the absence of the Tc compound be selected from the host cell that has been subjected to transfection. On the other hand, in the case of screening of the Tet-On system using an rtTA expression vector, the phrase "when the expression is induced" denotes, for example, a state in the presence of the Tc compound. Specifically, it is preferable that in step (B), cell lines that express the selection marker in the DNA-of-interest expression vector in the presence of the Tc compound be selected from the host cell that has been subjected to transfection.

Hereinafter, the screening method of the present invention is described in detail. However, the present invention is not limited to the following embodiments.

### <First Embodiment>

An example in which cotransfection is performed is described as a first embodiment.

In the present invention, it is preferable that both the aforementioned vectors be cotransfected because it allows the screening steps further to be shortened and simplified. In conventional methods, as described above, it is necessary that with respect to cell lines into which TA expression vectors have been transfected, assay of luciferase expression be performed using a control vector and thereby high expression cell lines be selected. Therefore, two-stage transfection was essential for transfecting the TA expression vector and the DNA-of-interest expression vector. However, according to the present invention, since the aforementioned luciferase assay is not required, one-stage transfection (cotransfection) also is possible. Furthermore, as described earlier, candidate cell lines highly likely to be the desired cell lines can be selected by only checking the expression of the selection marker of the DNA-of-interest expression vector.

### (A) Transfection of TA Expression Vector and DNA-of-interest Expression Vector

The TA expression vector and the DNA-of-interest expression vector are cotransfected into a target Nalm-6 host cell. Thus, the TA expression vector and the DNA-of-interest expression vector are transfected into the genome of the host cell. The vectors are transfected into the cell, for example, either in vivo or in vitro. The transfection method is not particularly limited and examples thereof include a calcium phosphate precipitation method, a DEAE-dextran transfection method, and an electroporation method. Besides these, examples thereof also include a method using, for example, a retroviral vector and an adenoviral vector, and this method also allows them to be transfected into a cell, for example, in vivo.

In the present invention, a tTA expression vector is used as the TA expression vector when cell lines of the "Tet-Off expression system" are screened, while an rtTA expression vector is used as the TA expression vector when cell lines of the "Tet-On expression system" are screened.

### (B) Selection of Vector Transfected Cell Lines

Subsequently, cell lines in which the selection marker of the DNA-of-interest expression vector is being expressed are selected from the host cell that has been subjected to cotransfection, while being considered as cell lines in which the DNA of interest also is being expressed at the same time. In the case of screening of the Tet-Off expression system using the tTA expression vector, it is preferable that, for example, the host cell be cultured in the absence of the Tc compound, and cell lines in which the selection marker in the DNA-of-interest expression vector has been expressed be selected. On the other hand, in the case of screening of the Tet-On expression system using the rtTA expression vector, it is preferable that, for example, the host cell be cultured in the presence of the Tc compound, and cell lines in which the selection marker in the DNA-of-interest expression vector has been expressed be selected. When the TA expression marker contains a selection marker, for example, cell lines may be selected in which both the selection marker of the TA expression vector and the selection marker of the DNA-of-interest expression vector have been expressed. The cell lines thus selected are candidate cell lines highly likely to be Tc inducible DNA expressing cell lines as described above.

The method of selecting the cell lines in which the selection marker has been expressed is not particularly limited and can be determined suitably according to the type of the selection marker. Specifically, when the selection marker is a drug-resistance marker, for example, a host cell that has been subjected to transfection is cultured in a culture medium containing a corresponding drug added thereto, and the cell lines thus grown can be selected as the transfected cell lines. The method of culturing the host cell is not particularly limited and can be determined suitably according to the type thereof (hereinafter, the same applies). In the case of screening of the Tet-On expression system, it is preferable that further a Tc compound be added to the culture medium. The amount of the Tc compound in the culture medium is, for example, in the range of 1 µg/ml to 3 µg/ml in the case of tetracycline.

As described above, the aforementioned TA expression vector further may contain a selection marker coding sequence. In this case, in step (B), cell lines in which the selection marker in the TA expression vector and the selection marker in the DNA-of-interest expression vector have been expressed may be selected from the host cell that has been treated in step (A).

### <Second Embodiment>

Next, an example in which sequential transfection is performed is described as a second embodiment.

### (A) Transfection of TA Expression Vector and DNA-of-interest Expression Vector

The target Nalm-6 host cell is subjected to transfection of the TA expression vector and subsequently to transfection of the DNA-of-interest expression vector. Thus, the TA expression vector and the DNA-of-interest expression vector are transfected into the genome of the host cell. The transfection method is not limited and is the same as described above (hereinafter, the same applies). In the case of the sequential transfection, in the aforementioned step (A), the order of transfection is not limited as long as after one vector is transfected, the other is transfected. For instance, after the DNA-of-interest expression vector is transfected, the TA expression vector may be transfected.

Furthermore, in the case of the sequential, transfection, for example, after the TA expression vector is transfected into a Nalm-6 host cell, cell lines into which the TA expression vector has been transfected are screened, and subsequently the DNA-of-interest expression vector may be transfected into the cell lines thus screened. The screening method is not particularly limited. For example, as described above, when the TA expression vector further contains a selection marker coding sequence, cell lines that express the selection marker can be selected from the host cell that have been subjected to the transfection. Furthermore, the cells thus selected may be subjected to luciferase assay using a control vector (for instance, pTRE2-Luc (trade name), manufactured by Clontech Laboratories, Inc.) in which the expression of the control gene is induced in the presence or absence of the Tc compound. As a result of the assay, selection of cell lines that express luciferase when expression is induced makes it possible, for example, to screen inducible DNA expressing cell lines at a higher probability.

### (B) Selection of Cell Lines with Vectors Transfected thereinto

Cell lines in which the selection marker of the DNA-of-interest expression vector has been expressed are selected as cell lines in which the DNA of interest also has been expressed at the same time, from the host cell that have been subjected to transfection. In the case of screening of the Tet-Off expression system using the tTA expression vector, it is preferable that, for example, the host cell be cultured in the absence of the Tc compound and cell lines in which the selection marker in the DNA-of-interest expression vector has been expressed be selected. Furthermore, in the case of screening of the Tet-On expression system using the rtTA expression vector, it is preferable that, for example, the host cell be cultured in the presence of the Tc compound and cell lines in which the selection marker in the DNA-of-interest expression vector has been expressed be selected. The cell lines thus selected are candidate cell lines highly likely to be Tc-inducible DNA expressing cells as described above. The aforementioned selection can be performed through selection of cell lines in which the selection marker in the DNA-of-interest expression vector has been expressed.

### <Third Embodiment>

The present invention further may include the following step (C) as the third embodiment:
(C) assaying expression of the DNA of interest in the presence and absence of the Tc compound with respect to the cell lines selected in the above-mentioned step (B).

As described above, by checking the expression of the DNA of interest in the presence/absence of the Tc compound with respect to the cell lines selected in the aforementioned step (B), it can be checked whether the cell lines are Tc inducible DNA expressing cell lines in which the expression of the DNA of interest can be regulated artificially, that is, so-called "Tc inducibility (Tc responsiveness)" can be checked. Accordingly, in the present embodiment, inducible DNA-of-interest expressing cell lines can be obtained from the candidate cell lines. Such a method makes it possible efficiently to obtain inducible DNA expressing cell lines in which the expression of the DNA of interest can be regulated artificially.

The aforementioned step (C) can be, for example, the following step (C1) or (C2). Step (C1) is a step in screening of the "Tet-Off expression system". In step (C1), cell lines that express the DNA of interest in the absence of the Tc compound and that lose the expression of the DNA of interest in the presence of the Tc compound are selected from the cell lines selected in the aforementioned step (B). Step (C2) is a step in screening of the "Tet-On expression system". In step (C2), cell lines that express the DNA of interest in the presence of the Tc compound and that lose the expression of the DNA of interest in the absence of the Tc compound are selected from the cell lines selected in the aforementioned step (B). The expression activity obtained when the DNA of interest is expressed is not particularly limited. For example, when the expression of an endogenous DNA of a parent strain (for instance, an untreated host strain) is taken as 100%, the expression activity is preferably 25 to 400%, more preferably 50 to 200%, and most preferably 100%. Preferably, it is expressed constitutively. Furthermore, for example, when the expression of an endogenous DNA of a parent strain (for instance, an untreated host strain) is taken as 100%, the expression activity that is obtained when the expression of the DNA of interest has been lost (suppressed) is preferably 50% or lower, more preferably 20% or lower, further preferably undetectable, and particularly preferably 0%. Preferably, the expression is suppressed constitutively.

In the assay, the type and amount of the Tc compound to be used and the method are not particularly limited and can be determined suitably based on conventionally known methods. Generally, the amount of the Tc compound that is added to the culture medium is, for example, in the range of 1 µg/ml to 3 µg/ml in the case of tetracycline. When screening of the "Tet-Off expression system" is performed, the expression of the DNA of interest is induced by, for example, cultivation in a culture medium that is free from the Tc compound, and subsequently the Tc compound is added to the culture medium to stop inducing the expression. Furthermore, when screening of the "Tet-On expression system" is performed, the expression of the DNA of interest is induced by, for example, cultivation in a culture medium containing the Tc compound, and the Tc compound is removed from the culture medium and thereby induction of the expression is stopped. Furthermore, it also is preferable that the inducibility be checked more closely by removing the Tc compound again or adding it again.

As described above, the type of the promoter is not limited and can be determined suitably according to the host cell. The method of selecting the promoter is not limited by any means and can be carried out, for example, as follows. That is, a TA expression vector with a CAG promoter arranged therein is prepared and is then transfected into a Nalm-6 host cell, and cell lines into which the TA expression vector has been transfected are then selected as described above. Thereafter, a control vector is transfected into the host cell thus selected. The control vector includes, for example, a control gene coding sequence and is a vector in which the expression of the control gene is induced in the presence or absence of the Tc compound. As described above, specific examples thereof include a vector containing a luciferase gene transfected as a control gene. With respect to the host cell into which the control vector has been transfected, the expression of the control gene in the presence or absence of the Tc compound is assayed. As a result, for example, when high expression (for instance, luciferase activity) of the control gene is exhibited in the case where expression is induced and the expression is suppressed in the case where expression is not induced, the promoter used can be selected as a preferable promoter. The method described above as an example is carried out for selecting a preferable promoter but is not an essential step in the present invention and thus does not limit the present invention.

### <Process of Producing Inducible DNA Expressing Cell Lines>

The process for producing an inducible DNA expressing cell line of the present invention is a process for producing an inducible DNA expressing cell line that allows the expression of a DNA of interest to be regulated depending on the presence or absence of a Tc compound, wherein the inducible DNA expressing cell line is obtained by a method of screening an inducible DNA expressing cell line of the present invention.

As long as the screening method of the present invention is included, other structures and conditions of the present invention are not limited by any means. According to the present invention, it becomes possible efficiently to obtain inducible DNA expressing cell lines (for instance, inducible gene expressing cell lines) that can regulate the expression of the DNA of interest (for instance, a gene of interest) artificially. Particularly, it is preferable that the present invention include a screening method of the present invention including the aforementioned step (C) in addition to the aforementioned steps (A) and (B). For example, the specific method and conditions are the same as those employed in the aforementioned screening method of the present invention.

The inducible DNA expressing cell lines obtained by the present invention allow the expression (transcription) of the DNA of interest to be switched ON/OFF depending on the presence or absence of the Tc compound. Therefore, the inducible DNA expressing cell lines of the present invention can be used as, for example, a tool for analyzing the function of a gene product. Specifically, for example, when a gene product is necessary for the growth or existence of a cell, the expression of the gene of interest is induced to a desired point of time and the expression of the gene of interest is allowed to be lost at a predetermined point of time. Thereafter, the change in phenotype between when expression was induced and when it was lost is checked and thereby it is possible to analyze the function of a protein, a product of the gene of interest.

### <Process for Producing Inducible DNA Knockout Cell Lines>

The process for producing an inducible DNA knockout cell line of the present invention is a process for producing an inducible DNA knockout cell line that allows the expression of a DNA of interest to be regulated depending on the presence or absence of a Tc compound, wherein the Tc compound is Tc or a Tc analog, and the process includes the following steps (a) to (c):
(a) before or after the following step (b), knocking out one allele of an endogenous DNA-of-interest sequence in a host cell which is a Nalm-6 cell,
(b) producing an inducible DNA expressing cell line by a production process of the present invention,
   where a DNA-of-interest expression vector is a vector that contains, as a DNA-of-interest sequence, an exogenous DNA sequence with the same function as that of the endogenous DNA-of-interest sequence of the host cell, and
(c) knocking out the other allele of the endogenous DNA-of-interest sequence in the cell line obtained in the aforementioned step (b).

The aforementioned step (b) corresponds to the screening method and the process for producing an inducible DNA expressing cell line of the present invention. Therefore, these steps can be performed, for example, in the same manners as described above. Particularly, it is preferable that the present invention include the screening method of the present invention including the aforementioned step (C) in addition to the aforementioned steps (A) and (B).

The DNA-of-interest expression vector of the present invention is a vector that contains the exogenous DNA sequence as the DNA-of-interest sequence. The exogenous sequence is not particularly limited and examples thereof include a DNA sequence with the same function as that of the endogenous DNA-of-interest sequence. Accordingly, it may be, for example, the same sequence as the endogenous DNA-of-interest sequence, or a sequence in which, for example, a few bases have been substituted, deleted, or added or a sequence with, for example, a homology of 70 to 100% as compared to the endogenous DNA-of-interest sequence. Preferably, it is the same DNA sequence as the endogenous DNA-of-interest sequence. Furthermore, the endogenous DNA-of-interest sequence may be, for example, a full length of endogenous gene of interest, a sequence including the full length, or a partial sequence of the full length. The endogenous DNA of interest to be subjected to knockout is not limited by any means.

In the present invention, the order of knocking out both of the alleles is not particularly limited. For example, one allele of the endogenous DNA-of-interest sequence in a host cell can be knocked out prior to the aforementioned step (b) and the other allele of the endogenous DNA-of-interest sequence in the resultant cell lines can be knocked out after the aforementioned step (b). Furthermore, both the alleles of the endogenous DNA-of-interest sequence in the resultant cell lines may be knocked out sequentially after the aforementioned step (b).

The method of knocking out alleles is not particularly limited. For example, a conventionally known method can be employed that utilizes a homologous recombination mechanism of the host cell. Specifically, there is a method in which a vector that has a structure with, for example, a drug-resistant coding sequence sandwiched between two polynucleotide sequences homologous to the endogenous DNA-of-interest sequence (for instance, an endogenous gene of interest) is prepared as a targeting vector, and then the targeting vector is transfected into a Nalm-6 host cell. When the targeting vector is transfected into the host cell, the homologous recombination mechanism of the host cell initiates a reaction of exchanging the endogenous DNA-of-interest sequence of the host cell for the drug-resistant sequence encoding the targeting vector. Accordingly, the endogenous DNA-of-interest sequence is deleted from the host cell that has been subjected to transfection and the host cell obtains the drug-resistance marker sequence derived from the targeting vector. Thus, the host cell in which the endogenous DNA-of-interest sequence has been knocked out can be selected as a cell that has obtained drug resistance.

The method of knocking out alleles is not particularly limited. Examples thereof include a method in which a targeting vector is transfected into a Nalm-6 host cell, and a homologous recombination mechanism of the host cell is used to knock out an endogenous sequence of interest through homologous recombination. Specific examples thereof include a method in which a polynucleotide sequence encoding a toxic protein such as dTA is added to a targeting vector (negative selection) and a method in which the homologous recombination mechanism or DNA repair mechanism of a host cell is artificially engineered transiently by genetic engineering. These methods are used individually or in combination to allow the endogenous DNA of interest (for instance, endogenous gene of interest) to be knocked out.

In the present invention, it is preferable that the process further includes the following step (d) after the aforementioned step (c):
(d) allowing the cell line obtained in the aforementioned step (c) to lose the expression of the exogenous DNA sequence in the presence or absence of the Tc compound.
This makes it possible to prepare cell lines that do not express not only the endogenous DNA of interest that is knocked out but also the exogenous DNA sequence that has been transfected.

When the inducible DNA expressing cell line produced in the aforementioned step (b) is a cell line that expresses the DNA of interest in the absence of the Tc compound and loses the expression of the DNA of interest in the presence of the Tc compound, it is preferable that the expression of the exogenous DNA sequence be allowed to be lost as follows. First, a cell line in which both alleles of the endogenous DNA-of-interest sequence (for instance, an endogenous gene of interest) have been knocked out is obtained in the absence of the Tc compound. This cell line expresses the exogenous DNA sequence (for instance, an exogenous gene) in the absence of the Tc compound. It is preferable that this cell line be allowed to be in the presence of the Tc compound in the aforementioned step (d) to lose the expression of the exogenous DNA sequence. On the other hand, when the inducible DNA expressing cell line produced in the aforementioned step (b) is a cell line that expresses the DNA of interest in the presence of the Tc compound and loses the expression of the DNA of interest in the absence of the Tc compound, it is preferable that the expression of the exogenous DNA be allowed to be lost as follows. First, a cell line in which both alleles of the endogenous DNA-of-interest sequence (for instance, an endogenous gene of interest) have been knocked out is obtained in the presence of the Tc compound. This cell line expresses the exogenous DNA sequence (for instance, an exogenous gene) in the presence of the Tc compound. Thereafter, it is preferable that this cell be allowed to be in the absence of the Tc compound in the aforementioned step (d) to lose the expression of the exogenous DNA sequence. The presence of the Tc compound can be obtained through, for example, addition of the Tc compound to a culture medium. On the other hand, the absence of the Tc compound can be obtained through, for example, not adding the Tc compound to a culture medium or removal of the Tc compound from the culture medium.

When the gene of interest is essential for the existence of a cell, there is a problem in that simple knockout results in mortality and thus no knockout cell line can be obtained. However, the process of the present invention makes it possible to control the expression of the gene of interest (exogenous gene) depending on the presence or absence of the Tc compound. Accordingly, when both alleles of the endogenous gene of interest are knocked out, with, for example, the cell being prevented from becoming lethal by maintaining the expression of the exogenous gene, a knockout cell line can be obtained without mortality. On the other hand, the knockout cell line thus obtained allows the expression of the exogenous gene of interest to be switched off depending on the presence or absence of the Tc compound. This allows the phenotype of the gene of interest to be analyzed. As described above, the present invention makes it possible to analyze the functions of gene products including so-called essential genes in cells that have not been studied well until now. Thus, the present invention can be said to be a very useful technique in the developments of drugs and clinical diagnosis kits and the fields of life science and clinical medicine. Furthermore, the inducible DNA knockout cell lines of the present invention can be used as, for example, negative controls for antibodies. In production of new antibodies, in order to check the effectiveness thereof, negative controls that do not express antigens are demanded. However, in the case where, for example, genes encoding the antigens merely are knocked out, there is a problem in that it results in lethality when the gene is essential for the growth thereof. Therefore, it is difficult to arrange such cell lines in the cell strain, with respect to which at least conventional methods do not function. On the other hand, according to the production process of the present invention, as described above, a gene of interest, i.e. a gene encoding an antigen is knocked out without causing a cell to be killed and the expression of the antigen is allowed to be lost at a desired time. Accordingly, the inducible gene knockout cell line of the present invention can be used as the aforementioned negative control when the expression of the antigen is allowed to be lost.

### <Process for Producing Altered DNA Inducible Knockout Cell Lines>

The process for producing an altered DNA inducible knockout cell line of the present invention is a process for producing an altered DNA knockout cell line in which an endogenous DNA has been knocked out and into which an altered DNA of the endogenous DNA whose expression can be regulated depending on the presence or absence of a Tc compound has been transfected, wherein the Tc compound is Tc or a Tc analog, and the process includes the following steps (1) to (n):
(l) before or after the following step (m), knocking out one allele of an endogenous DNA-of-interest sequence in a host cell which is a Nalm-6 cell,
(m) producing an inducible DNA expressing cell line by a production process of the present invention,
   where a DNA-of-interest expression vector is a vector that includes, as a DNA-of-interest sequence, an altered DNA sequence of the endogenous DNA-of-interest sequence of the host cell, and
(n) knocking out the other allele of the endogenous DNA-of-interest sequence in the cell line obtained in the aforementioned step (m).

The aforementioned step (m) corresponds to the screening method and the process for producing an inducible DNA expressing cell line of the present invention. Therefore, these steps can be carried out, for example, in the same manners as described above. Particularly, it is preferable that the present invention include the screening method of the present invention that includes the aforementioned step (C) in addition to the aforementioned steps (A) and (B).

The DNA-of-interest expression vector is identical to that described earlier except for containing the altered DNA sequence as a DNA-of-interest sequence. The altered DNA sequence is not particularly limited and examples thereof include a mutated DNA sequence of the endogenous DNA-of-interest sequence, a sequence obtained by fusing the endogenous DNA-of-interest sequence with a tag (a tag-fused DNA sequence), and functional homologous DNA sequences of other biological species. Examples of the endogenous DNA of interest include an endogenous gene, a sequence containing an endogenous gene, and a partial sequence of an endogenous gene. Examples of the mutated DNA sequence include a sequence encoding a mutated form (mutated protein) of a protein of interest (a protein that the endogenous gene codes). The type of the tag is not particularly limited and examples thereof include a sequence encoding a fluorescent protein such as a green fluorescence protein (GFP), a Flag tag, and an HA tag. The type of the endogenous DNA of interest to be subjected to knockout is not limited by any means.

In the present invention, the order of knocking out both of the alleles is not particularly limited. For example, one allele of the endogenous DNA-of-interest sequence in a Nalm-6 host cell can be knocked out prior to the aforementioned step (m) and the other allele of the endogenous DNA-of-interest sequence in the resultant cell lines can be knocked out after the aforementioned step (m). Furthermore, both the alleles of the endogenous DNA-of-interest sequence in the resultant cell lines may be knocked out sequentially after the aforementioned step (m).

In the present invention, when the expression of the transfected DNA is allowed to be lost after the aforementioned step (n), the following step (o) can be included:
(o) allowing the cell line obtained in the process (n) to lose the expression of the exogenous DNA sequence in the presence or absence of the Tc compound. This makes it possible to prepare cell lines that do not express not only the endogenous DNA-of-interest sequence (for instance, an endogenous gene) that is knocked out but also the altered DNA sequence (for instance, an altered gene) that has been transfected.

The aforementioned step (o) can be carried out in the same manner as in the aforementioned step (d) in the process for producing an inducible DNA knockout cell line of the present invention. Furthermore, alleles can be knocked out in the same manner as in the method described with respect to the process for producing an inducible DNA knockout cell line of the present invention.

The method of the present invention makes it possible, for example, to knock out both of the alleles of an endogenous DNA of interest without causing the cell to be lethal and to control the expressions of the altered exogenous DNA that has been transfected, depending on the presence or absence of the Tc compound. When an altered cell line into which an altered exogenous DNA has been transfected is used by such a method, for example, it is possible to analyze a protein domain whose function is unknown or to clarify the physiological significance of various protein modifications. Furthermore, according to the present invention, it also is possible to transfect sequences encoding proteins of interest fused with various tags as altered DNAs. This makes it possible, for example, to perform kinetic observation of a protein of interest in a viable cell or to purify the interacting factor of a protein of interest by using an antibody affinity column. Furthermore, the altered DNA inducible knockout cell lines obtained according to the present invention can be used as, for example, a negative control for an antibody. In conventional methods, there were the aforementioned problems in making negative controls. On the other hand, according to the production process of the present invention, a gene of interest, i.e. a gene encoding an antigen can be knocked out without causing the cell to be killed and the expression of the antigen is allowed to be lost at a desired time. Therefore, the inducible gene knockout cell lines of the present invention can be used as the aforementioned negative control by allowing the expression of the antigen to be lost. Furthermore, since the expression of a mutated protein of interest in which only a specific domain to be an antigen site or an amino acid has been modified can be induced, the inducible gene knockout cell lines of the present invention have a high value when an antibody that recognizes a specific site that has been subjected to posttranslational modification (for instance, phosphorylation, acetylation, methylation, or ubiquitination) is produced to be used as a negative control thereof. Accordingly, the present invention can be said to be a very useful technique in the developments of drugs and clinical diagnosis kits and the fields of life science and clinical medicine.

### [Examples]

Next, examples of the present invention are described. The present invention is not limited by the following examples.

### [Example 1]

With respect to Nalm-6 cells, screening cell lines of the "Tet-Off expression system" was performed.

### tTA Expression Vector

A CMV promoter of a pIRESneo3 vector was substituted by a pCAGGS vector-derived CAG promoter, and a pTet-Off vector-derived tTA was cloned into the multicloning site. The resultant vector is referred to as a pCAG-tTA-IRES-Neo vector. This was used as the tTA expression vector. FIG. 1(A) shows a part of the tTA expression vector. In FIG. 1(A), the pCAG and tTA denote a CAG promoter and a tTA coding sequence, respectively.

### Gene-of-Interest Expression Vector

A pMXs-IP vector-derived IRES-Puro sequence was cloned downstream of the multicloning site of a pTRE-Tight vector, and further a sequence that encodes NFH-hDDM1 was cloned into the multicloning site. The NFH-hDDM1 is a human DDM1 gene in which two epitope tags (a Flag tag and a HA tag) were added to the N-terminus (hereinafter, the same applies). The resultant vector is referred to as a pTRE-tight-NFH-hDDM1-IRES-Puro vector. This was used as a gene-of-interest expression vector. FIG. 1(B) shows a part of the pTRE-tight-NFH-hDDM1-IRES-Puro vector. In FIG. 1(B), Puro^{r} denotes a puromycin-resistant marker coding sequence, O denotes a sequence containing seven tet operator sequences arranged successively, and PminCMV denotes a minimum CMV promoter.

### Transfection of the gene of interest

First, the tTA expression vector was transfected into Nalm-6 cells. The tTA expression vector was transfected by the following method using a gene transfection system (Trade Name: Nucleofector (registered trademark), manufactured by Amaxa) and a gene transfection reagent exclusively for the system.

Nalm-6 cells (about 2 × 10⁶) in the exponential growth phase were prepared, and the aforementioned gene transfection reagent and 2 µg of the aforementioned tTA expression vector were added thereto. This was treated using the gene transfection system. After completion of the treatment, 4 ml of ES culture medium that had been warmed to 37°C was added to the cells, which then was allowed to stand inside a CO₂ incubator at 37°C for 24 hours.

The cells that had been allowed to stand for 24 hours were diluted with an ES culture medium containing G418 whose final concentration was 1.4 mg/ml so that the cell concentration was about 5 × 10³ cells/ml. This cell suspension was plated into a 96-well plate (200 µl of cell suspension/well, 1×10³ cells/well). This plate was allowed to stand inside a CO₂ incubator at 37°C for two weeks. After two weeks, cell lines that exhibited resistance to G418 were obtained as tTA stable cell lines.

Subsequently, the resultant cloned cell lines (tTA stable cell lines) were transfected with a pTRE-Tight-Luc vector (trade name, manufactured by Clontech Laboratories, Inc.). This was cultured for 24 hours. In this case, the method of vector transfection and the culture method are the same as those described above except for using a drug-free ES culture medium.

A cell extract was prepared from the resultant cultured cells by a conventionally known method and luciferase activity corresponding to 2 ×10⁵ cells was then measured. The promoter that induces the expression of a tTA (or expression of an rtTA) is not particularly limited and can be selected according to the cell strain to be used as a host cell. Then, the promoter that induced the expression of a tTA in the Nalm-6 cells was tested by the luciferase assay. For the measurement of the luciferase activity, Luciferase Assay System (trade name, manufactured by Promega) was used as a reagent, and Lumat LB 9507 (trade name, manufactured by BERTHOLD TECHNOLOGIES) was used as a luminometer. The luciferase activity was measured using only the above-mentioned reagent free from the cell extract and the value thus obtained was used as background. This result is indicated below in Table 1. As indicated in Table 1, all the resultant cell lines exhibited high luciferase activity. From this result, it was proved that the CAG promoter was effective for inducing the expression of the tTA (or rtTA) in the Nalm-6 cells.

**[Table 1]**

| Cell lines | Luciferase Activity (Value displayed on Luminometer) |
|---|---|
| Control | 68 |
| CAG clone 2 | 2,026,095 |
| CAG clone 4 | 1,352,896 |
| CAG clone 5 | 1,616,378 |
| CAG clone 6 | 1,934,572 |
| CAG clone 7 | 1,806,146 |
| CAG clone 8 | 2,126,595 |
| CAG clone 9 | 1,303,658 |
| CAG clone 10 | 1,786,661 |
| CAG clone 11 | 1,712,063 |
| CAG clone 12 | 1,147,917 |
| CAG clone 14 | 1,425,979 |

A gene-of-interest expression vector (pTRE-tight-NFH-hDDM1-IRES-Puro vector) obtained by cloning a gene of interest was transfected into the cell line (CAG clone 8 indicated in Table 1) with the highest luciferase activity among the cell lines by the same method as that used in the case of the tTA expression vector. The cell line that had been subjected to transfection further was cultured in an ES culture medium containing puromycin whose final concentration was 0.2 µg/ml, at 37°C for two weeks. The puromycin-resistant cell lines thus grown were selected as cell lines into which the gene-of-interest expression vector had been transfected.

With respect to 11 arbitrary cell lines among the puromycin-resistant cell lines thus selected, the presence or absence of protein expression of each gene of interest was checked by common Western blot using an antibody.

First, a lysate prepared from each of the aforementioned cell lines was electrophoresed using 7% by weight of polyacrylamide gel, and a protein contained in the gel thus electrophoresed was transferred to a nitrocellulose membrane. A primary antibody was added to the nitrocellulose membrane and an antigen-antibody reaction was performed. Thereafter, a secondary antibody further was added thereto, and thereby an antigen-antibody reaction was performed between the primary antibody that had been bound to an antigen (HA epitope added to the hDDM1 protein) and the secondary antibody added. Thereafter, the nitrocellulose membrane was brought into contact with a detection reagent, and the protein of interest (NFH-HDDM1 protein) then was detected, with an X-ray film being exposed with chemiluminescence. In this case, in the electrophoresis, in order to indicate that an equal amount of protein was electrophoresed in each lane, a background signal that was generated by an α-HA antibody was detected together as a loading control. This result is shown in FIG. 2. FIG. 2 is a photograph showing the expression (protein expression) of the NFH-hDDM1 genes in a plurality of puromycin-resistant cell lines. As shown in FIG. 2, with respect to 10 out of the 11 puromycin-resistant cell lines, the expression of NFH-hDDM1 was observed. From this result, it was proved that cell lines transfected with a gene of interest and expressing the gene of interest in response to expression induction can be obtained at a very high rate of 10/11.

### Check of Tc Inducibility

With respect to the puromycin-resistant cell line of Clone No. 5 shown in FIG. 2, suppression of the expression of the gene of interest (suppression of the expression of an NFH-hDDM1 protein) due to addition of Tc and reexpression of the gene of interest (reexpression of the NFH-hDDM1 protein) through Tc removal were checked.

First, the puromycin-resistant line was cultured (37°C) in an ES culture medium containing Tc that had been added in such a manner as to have a final concentration of 1 µg/ml, for predetermined periods of time (0, 3, 6, 12, 24, and 48 hours after Tc addition). Subsequently, removal of the Tc-containing ES culture medium by centrifugation and washing of cultured cells with a new Tc-free ES culture medium was repeated twice to remove Tc from the culture medium. Furthermore, this was cultured in the Tc-free ES culture medium for predetermined periods of time (0, 3, 6, 12, 24, and 48 hours after Tc removal). In such a series of culture processes, the expression of the NFH-hDDM1 protein in the puromycin-resistant cell line was checked by the same indirect detection method (Western blotting) as that described above. These results are shown in FIG. 3. FIG. 3 shows photographs that indicate the expression (protein expression) of the NFH-hDDM1 gene in the puromycin-resistant cell line; FIG. 3(a) shows the result that indicates suppression of the expression after Tc addition while FIG. 3(b) shows the result that indicates reexpression after Tc removal.

As shown in FIG. 3(a), in the puromycin-resistant cell line with a gene of interest (NFH-hDDM1 gene) transfected thereinto, the level of expression of the NFH-hDDM1 was suppressed over time after Tc addition. After 48 hours, no band that indicated the hDDM1 protein was observed. Furthermore, as shown in FIG. 3(b), the expression of the NFH-hDDM1 was observed again over time through removal of Tc in the cell line in which the expression of the NFH-hDDM1 gene had been suppressed by Tc. This proved that this example made the following possible: the expression of the tTA, transfection of the gene of interest, and control of the expression of the gene of interest depending on the presence/absence of Tc.

### [Example 2]

With respect to Nalm-6 cells, screening cell lines of the "Tet-On expression system" was performed. This example was performed in the same manner as in Example 1 unless otherwise particularly indicated.

### Construction of rtTA Expression Vector

A pTet-On vector-derived rtTA was cloned into a pCAGGS vector. The resultant vector was used as an rtTA expression vector.

### Gene-of-interest expression vector

An NFH-hDDM1 gene was cloned into a multicloning site of a pTRE-tight-IRES-Neo vector. The resultant vector was used as a gene expression vector.

### Transfection of Vectors

The rtTA expression vector and the gene-of-interest expression vector were cotransfected into Nalm-6 cells. The method of transfecting the vectors is the same as that employed in Example 1. The Nalm-6 cells that had been subjected to transfection was cultured in an ES culture medium containing neomycin (with a final concentration of 1.2 mg/ml) and Tc (with a final concentration of 2 µg/ml) and thereby a plurality of neomycin-resistant cell line were obtained.

### Check of Tc Inducibility

### (1) In the presence of TC

With respect to 12 arbitrary cell lines selected from the resultant neomycin-resistant cell lines, the expressions of the NFH-hDDM1 protein and endogenous hDDM1 protein were checked by Western blotting in the same manner as in Example 1. These results are shown in FIG. 4. FIG. 4 shows the expressions (protein expressions) of the NFH-hDDM1 genes in the neomycin-resistant cell lines. As shown in FIG. 4, in Example 2, four cell lines out of 12 neomycin-resistant cell line expressed the NFH-hDDM1 protein. From this result, it was proved that this example made it possible to obtain cell lines capable of expressing a gene of interest at a very high rate.

### (2) Tc Removal and Tc Readdition

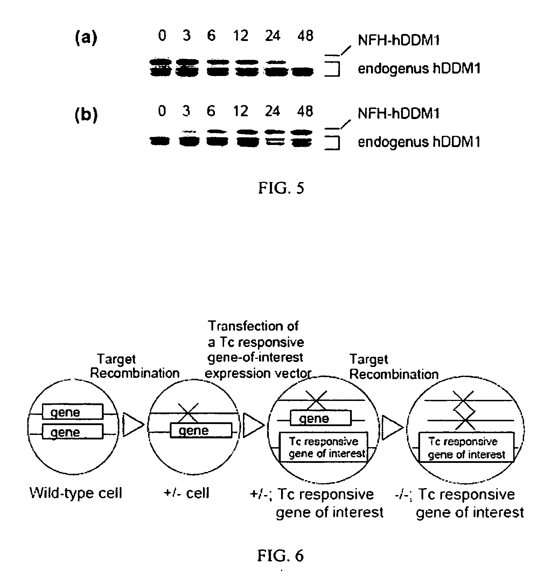

Subsequently, with respect to the neomycin-resistant cell lines that had expressed the NFH-hDDM1 protein, the loss of expression due to Tc removal and reexpression due to Tc readdition were checked. Specifically, with respect to each neomycin-resistant cell line that had expressed the NFH-hDDM1 protein, Tc was removed from the culture medium, and Tc was added to the culture medium again in such a manner as to have a final concentration of 2 µg/ml after a lapse of 48 hours after removal in the same manner as in Example 1. In such a series of culture processes, the expression of the NFH-hDDM1 protein in the cell line was checked by the aforementioned indirect detection method. These results are shown in FIG. 5. FIG. 5 shows the expression (protein expression) of the NFH-hDDM1 gene in the neomycin-resistant cell line; FIG. 5(a) shows the result that indicates suppression of the expression after Tc removal while FIG. 5(b) shows the result that indicates reexpression when Tc was readded. The numerals in both the figures indicate the periods of time that have elapsed after start of Tc removal and after Tc readdition. As a result, as shown in FIG. 5(a), the expression of NFH-hDDM1 was lost after 48 hours after Tc removal. Furthermore, as shown in FIG. 5(b), when Tc was added again, the expression of NFH-hDDM1 was observed three hours later. Thus, it was proved that similarly in the Tet-On expression system, cell lines that express gene of interest in an inducible manner were able to be obtained.

### [Example 3]

### tTA Expression Vector

A pTet-Off vector-derived tTA was cloned into a pCAGGS vector. The resultant vector was used as a tTA expression vector.

### Gene-of-interest Expression Vector

An NFH-hDDM1 gene was cloned into a multicloning site of a pTRE-tight-IRES-Neo vector. The resultant vector was used as a gene expression vector.

A Tc-inducible knockout cell line was produced. FIG. 6 shows the outline of the process for producing the Tc-inducible knockout cell line.

As shown in FIG. 6, first, one allele of an endogenous gene of interest of a wild-type Nalm-6 cell (wild-type cell) was disrupted by a conventionally known method and thereby a hetero strain (+/-) was produced. Subsequently, the Tc responsive gene -of-interest expression vector and the tTA expression vector were transfected into the hetero strain (+/-; Tc responsive gene of interest). In this case, the cell that had been subjected to transfection was cultured in an ES culture medium (Tc free) containing a drug, and then drug-resistant cell line clones were selected. Subsequently, the second allele of endogenous GOI loci was disrupted by a conventionally known method. Thus, a Tc-inducible conditional knockout cell line was produced (-/-; Tc responsive gene of interest). The drug contained in the culture medium was puromycin.

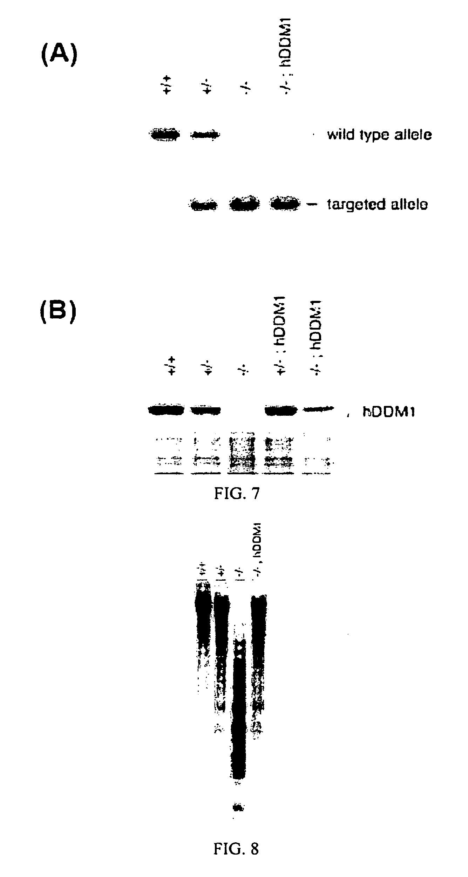

With respect to the wild-type cell line, hetero line (+/-), hetero line (+/-; hDDM1) that expressed hDDM1 with Tc responsiveness, and conditional knockout cell line (-/-; hDDM1), the genotypes of endogenous hDDM1 gene loci were checked by Southern blot. This result is shown in FIG. 7(A). FIG. 7(A) is an autoradiography that shows the genotype of each cell line. As shown in FIG. 7(A), both alleles had been destroyed in the finally-obtained conditional knockout cell lines. Furthermore, with respect to these cell lines, the expression of the hDDM1 protein was checked by Western blot. This result is shown in FIG. 7(B). FIG. 7(B) shows the expression of the hDDM1 protein of each cell line. As shown in FIG. 7(B), both alleles of the endogenous hDDM1 gene had been disrupted in each of the finally-obtained conditional knockout cell lines, but the expression of the hDDM1 protein due to transfection of vectors was observed. Furthermore, genomes purified from those cell lines each were cleaved by a restriction enzyme that was susceptible to DNA methylation and Southern blot was then performed using a satellite 2 probe. This result is shown in FIG. 8. FIG. 8 is an autoradiograph that shows the cleavage pattern of the purified genome of each cell line. As shown in FIG. 8, since the genome of the finally obtained conditional knockout cell line exhibited the same behavior as that of the wild-type or hetero line (+/-), it was proved that the hDDM1 that had been transfected was functional and DNA methylation was maintained. Thus, it was proved that the method of this example made it possible to obtain Tc-inducible knockout cell lines, in each of which, for example, a gene of interest was knocked out without causing the cell to be killed by knocking out both alleles and expression was able to be induced in the absence of Tc. [Example 4] An altered gene harboring knockout cell line was produced in which an endogenous gene was knocked out and was substituted by an altered gene capable of regulating expression depending on the presence or absence of a Tc compound.

First, one allele of an endogenous hDDM1 gene of a wild-type Nalm-6 cell (wild-type cell) was disrupted by a conventionally known method and thereby a hetero line (+/-) was produced. Subsequently, a gene expression vector, into which a hDDM1 gene (NFH-hDDM1 gene) with an HA epitope tag added thereto had been inserted, and the tTA expression vector used in Example 3 were transfected into the hetero clone (+/-; NFH-hDDM1. The gene expression vector was produced through cloning of the NFH-hDDM1 gene into a multicloning site of the pTRE-tight-IRES-Puro vector produced in Example 1. In this case, the cell that had been subjected to the transfection was cultured in an ES culture medium (Tc free) containing puromycin in the same manner as in Example 1 and then a puromycin-resistant cell line was selected. This cell line contained a Tc responsive (Tet-Off) NFH-hDDM1 gene transfected thereinto. Subsequently, the endogenous hDDM1 gene of the other allele was disrupted by a conventionally known method, and thereby an altered gene harboring knockout cell line, in which the endogenous gene had been substituted by an altered gene, was produced (-/-; NFH-hDDM1).

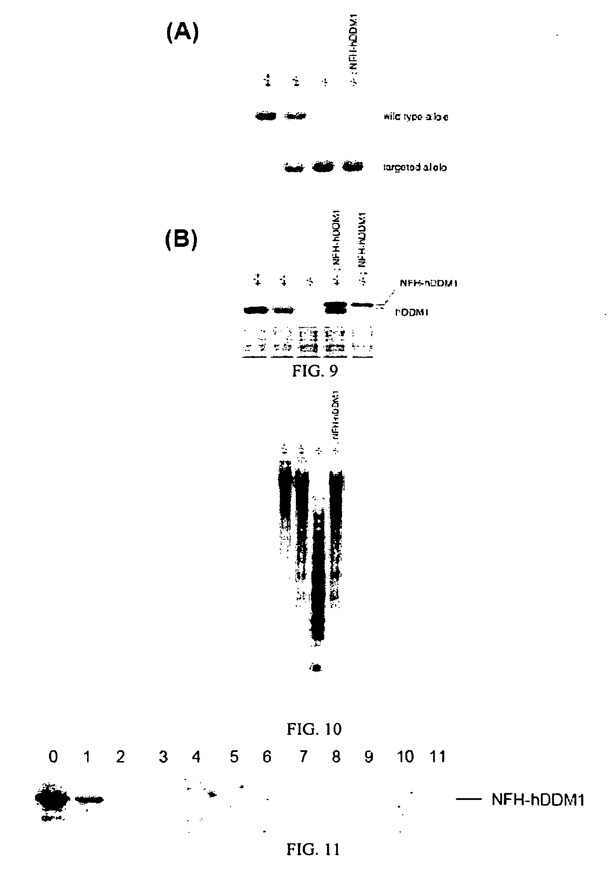

With respect to the wild-type cell line, hetero strain (+/-), hetero line (+/-; NFH-hDDM1) that expressed NFH-hDDM1 with Tc responsiveness, and altered gene harboring knockout cell line (-/-; NFH-hDDM1), the genotype of endogenous hDDM1 gene was checked by Southern blot. This result is shown in FIG. 9(A). FIG. 9(A) is an autoradiograph that shows the genotype of each cell line. As shown in FIG. 9(A), both alleles had been destroyed in the finally obtained altered gene harboring knockout cell lines. Furthermore, with respect to these cell lines, the expression of the NFH-HDDM1 protein was checked by Western blot. This result is shown in FIG. 9(B). FIG. 9(B) shows the expression of the endogenous hDDM1 protein and NFH-hDDM1 protein of each line. As shown in FIG. 9(B), both alleles of the endogenous hDDM1 gene had been disrupted in each of the finally obtained altered gene knockout cell lines, but the expression of the NFH-hDDM1 protein due to transfection of vectors was observed. Furthermore, genomes purified from those cell lines each were cleaved by a restriction enzyme that was susceptible to DNA methylation and Southern blot was then performed using a satellite 2 probe. This result is shown in FIG. 10. FIG. 10 is an autoradiograph that shows the cleavage pattern of the purified genome of each line. As shown in FIG. 10, since the genome of the finally obtained altered gene harboring knockout cell line exhibited the same behavior as that of the wild-type or hetero line (+/-), it was proved that the protein derived from the NFH-hDDM1 gene that had been transfected had a function of maintaining DNA methylation of the endogenous hDDM1 gene. Thus, the method of this example makes it possible to easily obtain cell lines, in each of which the endogenous gene has been substituted by the altered gene.

### [Example 5]

The applicability of Tc inducible gene knockout cells as an antibody verification tool was checked.

In the same manner as in Example 4, an altered gene harboring knockout line (-/-; NFH-hDDM1) was produced, in which an endogenous hDDM1 gene had been knocked out and into which Tc responsive (Tet-Off) altered hDDM1 gene (NFH-hDDM1 gene) had been transfected. The altered gene harboring knockout cell line was cultured in an ES culture medium containing Tc that had been added thereto in such a manner as to have a final concentration of 2 µg/ml, and cultured cells were collected every 24 hours after Tc addition. With respect to the cell lines thus collected, Western blot was carried out using an α-hDDM1 antibody. This result is shown in FIG. 11. FIG. 11 is a photograph showing the expression of the NFH-hDDM1 protein in the above-mentioned cell line. Furthermore, in FIG.11, the numerals indicated in the upper part of the photograph denote the number of days after Tc addition. As shown in FIG. 11, the NFH-hDDM1 protein was detected on Day 0, with no Tc being added, but it was not detected after Day 3. From this result, it is proved that the α-hDDM1 antibody is applicable to Western blot.

As described above, it was proved that the Tc inducible gene knockout cell lines and altered gene harboring knockout cell lines obtained according to the present invention were useful as tools for judging whether, for example, antibodies that had been produced or purchased were applicable to tests such as Western blot, immunostaining, or immunoprecipitation. Furthermore, antibody drugs are attracting attention as cancer drugs recently, and the Tc inducible gene knockout cell lines are considered to be very useful as tools for verifying the specificities thereof.

### Industrial Applicability

The present invention makes it possible to screen cells that cannot control the expression of DNA of interest depending on the presence or absence of a Tc compound by using the aforementioned TA expression vector and DNA-of-interest expression vector and merely checking the expression of a selection marker in the DNA-of-interest expression vector. This makes it possible to screen candidate cell lines with very high probability of being Tc inducible DNA-of-interest expressing cell lines. Furthermore, since the cell lines that are selected according to the present invention are highly probable candidate cell lines, it is sufficient to check the control of the expression of DNA of interest depending on the presence or absence of a Tc compound ultimately with respect to, for example, only the candidate cell lines. Accordingly, as compared to conventional methods, the whole method of screening Tc inducible DNA-of-interest expressing cell lines can be performed easily. In this manner, since the present invention allows screening to be performed easily and efficiently, it becomes possible to obtain Tc inducible DNA-of-interest expressing cell lines in, for example, a shorter period of time as compared to conventional cases. Furthermore, similarly with respect to, for example, hematopoietic cells, from which it has been difficult to obtain Tc inducible DNA-of interest expressing cell lines stochastically, it can be said that the possibility of obtaining desired cell lines has improved. Thus, the present invention makes it possible to, for example, analyze the functions of gene products in cells that have not been studied sufficiently until now, and therefore the present invention can be said to be a very useful technique in the developments of drugs and clinical diagnosis kits and the fields of life science and clinical medicine. The screening method of the present invention also can be considered to be, for example, a method of screening candidate cell lines with a high probability of being Tc inducible DNA-of-interest expressing cell lines.

### [Sequence Table]

TF07035-01.ST25.txt

## Claims

1. A method of screening an inducible DNA expressing cell line that allows expression of a DNA of interest to be regulated depending on the presence or absence of a tetracycline compound,
wherein the tetracycline compound is tetracycline or a tetracycline analog, and
the method comprises the following processes (A) and (B),
(A) transfecting a transactivator expression vector and a DNA-of-interest expression vector into a host cell, wherein the host cell is a Nalm-6 cell,
where the transactivator expression vector includes:
a polynucleotide sequence encoding a transactivator and a CAG promoter sequence that controls transcription of the polynucleotide sequence encoding a transactivator, with the polynucleotide sequence encoding a transactivator being arranged under control of the CAG promoter sequence,
the transactivator is a protein that is switched to be bound or unbound to a tet operator sequence depending on the presence or absence of the tetracycline compound, and the transactivator is a fusion protein containing a Tet repressor and a transcriptional activation domain or a fusion protein containing a reverse Tet repressor and a transcriptional activation domain, and
the DNA-of-interest expression vector includes:
a tet operator sequence, a DNA-of-interest sequence, a promoter sequence that controls transcription of the DNA-of-interest sequence, a bicistronic regulatory sequence, and a polynucleotide sequence encoding a selection marker, with the bicistronic regulatory sequence being arranged between the DNA-of-interest sequence and the polynucleotide sequence encoding a selection marker under control of the tet operator sequence and the promoter sequence, and
(B) selecting cell lines, in each of which the selection marker in the DNA-of-interest expression vector has been expressed, from the host cell that has been subjected to transfection of the transactivator expression vector and the DNA-of-interest expression vector.

2. The method according to claim 1, wherein in the process (A), the transactivator expression vector and the DNA-of-interest expression vector are cotransfected into the host cell.

3. The method according to claim 1, wherein
I) the transactivator is a fusion protein containing a Tet repressor and a transcriptional activation domain,
the Tet repressor is polypeptide that is bound to the tet operator sequence in the absence of the tetracycline compound and that is not bound to the tet operator sequence in the presence of the tetracycline compound, and
in the process (B), cell lines are selected, each of which expresses the selection marker in the DNA-of-interest expression vector in the absence of the tetracycline compound, from the host cell that has been subjected to transfection; or
II) the transactivator is a fusion protein containing a reverse Tet repressor and a transcriptional activation domain,
the reverse Tet repressor is polypeptide that is bound to the tet operator sequence in the presence of the tetracycline compound and that is not bound to the tet operator sequence in the absence of the tetracycline compound, and
in the process (B), cell lines are selected, each of which expresses the selection marker in the DNA-of-interest expression vector in the presence of the tetracycline compound, from the host cell that has been subjected to transfection.

4. The method according to claim 1, wherein the selection marker is a drug resistance selection marker.

5. The method according to claim 1, further comprising the following process (C):
(C) assaying the expression of the DNA of interest in the presence and absence of the tetracycline compound with respect to the cell lines selected in the process (B).

6. The method according to claim 5, wherein the process (C) is either of the following processes (C1) or (C2):
(C1) selecting a cell line that expresses the DNA of interest in the absence of the tetracycline compound and that loses the expression of the DNA of interest in the presence of the tetracycline compound, from the cell lines selected in the process (B), and
(C2) selecting a cell line that expresses the DNA of interest in the presence of the tetracycline compound and that loses the expression of the DNA of interest in the absence of the tetracycline compound, from the cell lines selected in the process (B).

7. A process for producing an inducible DNA of interest expressing cell line that allows the expression of a DNA of interest to be regulated depending on the presence or absence of a tetracycline compound,
wherein the inducible DNA of interest expressing cell line is obtained by a method of screening an inducible DNA of interest expressing cell line according to claim 1.

8. A process for producing an inducible DNA knockout cell line into which a DNA of interest whose expression can be regulated in the presence or absence of a tetracycline compound has been transfected,
wherein the tetracycline compound is tetracycline or a tetracycline analog, and
the process comprises the following processes (a) to (c):
(a) before or after the following process (b), knocking out one allele of an endogenous DNA-of-interest sequence in a host cell, wherein the host cell is a Nalm-6 cell,
(b) producing an inducible DNA-of-interest expressing cell line by a process according to claim 7,
where a DNA-of-interest expression vector is a vector that includes, as a DNA-of-interest sequence, an exogenous DNA sequence with the same function as that of the endogenous DNA-of-interest sequence of the host cell, and
(c) knocking out the other allele of the endogenous DNA-of-interest sequence in the cell line obtained in the process (b).

9. The process according to claim 8, further comprising the following process (d) after the process (c):
(d) allowing the cell line obtained in the process (c) to lose the expression of the exogenous DNA sequence in the presence or absence of the tetracycline compound;
wherein optionally
I) the inducible DNA expressing cell line is a cell line that expresses the DNA of interest in the absence of the tetracycline compound and that loses the expression of the DNA of interest in the presence of the tetracycline compound, and
in the process (d), the cell line obtained in the process (c) is allowed to lose the expression of the exogenous DNA sequence in the presence of the tetracycline compound; or
II) the inducible DNA expressing cell line is a cell line that expresses the DNA of interest in the presence of the tetracycline compound and that loses the expression of the DNA of interest in the absence of the tetracycline compound, and
in the process (d), the cell line obtained in the process (c) is allowed to lose the expression of the exogenous DNA sequence in the absence of the tetracycline compound.

10. A process for producing an altered DNA knockout cell line in which an endogenous DNA has been knocked out and into which an altered DNA whose expression can be regulated depending on the presence or absence of a tetracycline compound has been transfected,
wherein the tetracycline compound is tetracycline or a tetracycline analog, and
the process comprises the following processes (1) to (n):
(l) before or after the following process (m), knocking out one allele of an endogenous DNA-of interest sequence in a host cell, wherein the host cell is a Nalm-6 cell,
(m) producing an inducible DNA expressing cell line by a process according to claim 7,
where a DNA-of-interest expression vector is a vector that includes, as a DNA-of-interest sequence, an altered DNA sequence of the endogenous DNA-of-interest sequence of the host cell, and
(n) knocking out the other allele of the endogenous DNA-of-interest sequence in the cell line obtained in the process (m).

11. The process according to claim 10, wherein the altered DNA sequence has a mutated sequence of the endogenous DNA sequence or a sequence of a homolog of a different biological species from that of the endogenous DNA sequence, or a tag-fused sequence obtained by fusing a tag to the endogenous DNA sequence.

12. The process according to claim 11, further comprising the following process (o) after the process (n),
(o) allowing the cell line obtained in the process (n) to lose the expression of the altered DNA sequence in the presence or absence of the tetracycline compound;
wherein optionally
I) the inducible DNA expressing cell line is a cell line that expresses a DNA of interest in the absence of the tetracycline compound and that loses the expression of the DNA of interest in the presence of the tetracycline compound, and
in the process (o), the cell line obtained in the process (n) is allowed to lose the expression of the altered DNA sequence in the presence of the tetracycline compound; or
II) the inducible DNA expressing cell line is a cell line that expresses a DNA of interest in the presence of the tetracycline compound and that loses expression of the DNA of interest in the absence of the tetracycline compound, and
in the process (o), the cell line obtained in the process (n) is allowed to lose the expression of the altered DNA sequence in the absence of the tetracycline compound.

## Patentansprüche

1. Verfahren zum Screening einer induzierbare DNA exprimierenden Zelllinie, die erlaubt, dass die Expression einer DNA von Interesse in Abhängigkeit von der Anwesenheit oder Abwesenheit einer Tetracyclin-Verbinding reguliert wird,
wobei die Tetracyclin-Verbindung Tetracyclin oder ein Tetracyclin-Analogon ist und
das Verfahren die folgenden Prozesse (A) und (B) umfasst,
(A) Transfizieren eines Transaktivator-Expressionsvektors und eines DNA-von-Interesse-Expressionsvektors in eine Wirtszelle, wobei die Wirtszelle eine Nalm-6-Zelle ist,
wobei der Transaktivator-Expressionsvektor umfasst:
eine Polynukleotidsequenz, die einen Transaktivator codiert, und eine CAG-Promotorsequenz, die die Transkription der Polynukleotidsequenz, die den Transaktivator codiert, kontrolliert, wobei die Polynukleotidsequenz, die den Transaktivator codiert, unter der Kontrolle der CAG-Promotorsequenz angeordnet ist,
der Transaktivator ein Protein ist, das in Abhängigkeit von der Anwesenheit oder Abwesenheit der Tetracyclin-Verbindung geschaltet wird, so dass es an eine tet-Operator-Sequenz gebunden ist oder ungebunden ist, und der Transaktivator ein Fusionsprotein ist, das einen Tet-Repressor und eine Transkriptionsaktivierungsdomäne enthält, oder ein Fusionsprotein ist, das einen reversen Tet-Repressor und eine Transkriptionsaktivierungsdomäne enthält, und
der DNA-von-Interesse-Expressionsvektor umfasst:
eine tet-Operator-Sequenz, eine DNA-von-Interesse-Sequenz, eine Promotorsequenz, die die Transkription der DNA-von-Interesse-Sequenz kontrolliert, eine bicistronische Regulatorsequenz und eine Polynukleotidsequenz, die einen Selektionsmarker codiert, wobei die bicistronische Regulatorsequenz zwischen der DNA-von-Interesse-Sequenz und der Polynukleotidsequenz, die einen Selektionsmarker codiert, unter der Kontrolle der tet-Operatursequenz und der Promotorsequenz angeordnet ist, und
(B) Selektieren von Zelllinien, wobei in jeder von diesen der Selektionsmarker in den DNA-von-Interesse-Expressionsvektor aus der Wirtszelle exprimiert wurde, welche einer Transfektion des Transaktivator-Expressionsvektor und des DNA-von-Interesse-Expressionsvektors unterworfen wurde.

2. Verfahren nach Anspruch 1, wobei in dem Prozess (A) der Transaktivator-Expressionsvektor und der DNA-von-Interesse-Expressionsvektor in die Wirtszelle cotransfiziert werden.

3. Verfahren nach Anspruch 1, wobei
I) der Transaktivator ein Fusionsprotein ist, das einen Tet-Repressor und eine Transkriptionsaktivierungsdomäne enthält,
der Tet-Repressor ein Polypeptid ist, das an die tet-Operator-Sequenz in Abwesenheit der Tetracyclin-Verbindung gebunden ist und das in Anwesenheit der Tetracyclin-Verbindung nicht an die tet-Operator-Sequenz gebunden ist, und
im Prozess (B) Zelllinien selektiert werden, von denen jede den Selektionsmarker in dem DNA-von-Interesse-Expressionsvektor in Abwesenheit der Tetracyclin-Verbindung aus der Wirtszelle, die einer Transfektion unterzogen wurde, exprimiert, oder
II) der Transaktivator ein Fusionsprotein ist, das einen reversen Tet-Repressor und eine Transkriptionsaktivierungsdomäne enthält,
wobei der reverse Tet-Repressor ein Polypeptid ist, das in Anwesenheit der Tetracyclin-Verbindung an die tet-Operator-Sequenz gebunden ist und das in Abwesenheit der Tetracyclin-Verbindung nicht an die tet-Operator-Sequenz gebunden ist, und
im Prozess (B) Zelllinien selektiert werden, von denen jede den Selektionsmarker in dem DNA-von-Interesse-Expressionsvektor in Gegenwart der Tetracyclin-Verbindung aus der Wirtszelle, die einer Transfektion unterzogen wurde, exprimiert.

4. Verfahren nach Anspruch 1, wobei der Selektionsmarker ein Wirkstoffresistenz-Selektionsmarker ist.

5. Verfahren nach Anspruch 1, das außerdem den folgenden Prozess (C) umfasst:
(C) Analysieren der Expression der DNA von Interesse in Anwesenheit und Abwesenheit der Tetracyclin-Verbindung für die im Prozess (B) selektierten Zelllinien.

6. Verfahren nach Anspruch 5, wobei der Prozess (C) einer der folgenden Prozesse (C1) oder (C2) ist:
(C1) Selektieren einer Zelllinie, die die DNA von Interesse in Abwesenheit der Tetracyclin-Verbindung exprimiert und die die Expression der DNA-von-Interesse in Gegenwart der Tetracyclin-Verbindung verliert, aus den im Prozess (B) selektierten Zelllinien und
(C2) Selektieren einer Zelllinie, die die DNA von Interesse in Gegenwart der Tetracyclin-Verbindung exprimiert und die die Expression der DNA-von-Interesse in Abwesenheit der Tetracyclin-Verbindung verliert, aus den im Prozess (B) selektierten Zelllinien.

7. Verfahren zur Herstellung einer induzierbare DNA-von-Interesse exprimierenden Zelllinie, die erlaubt, dass die Expression einer DNA von Interesse in Abhängigkeit von der Anwesenheit oder Abwesenheit einer Tetracyclin-Verbindung reguliert wird,
wobei die induzierbare DNA von Interesse exprimierende Zelllinie durch ein Verfahren zum Screening einer induzierbare DNA von Interesse exprimierenden Zelllinie nach Anspruch 1 erhalten wird.

8. Verfahren zur Herstellung einer induzierbare DNA-Knockout-Zelllinie, in welche eine DNA von Interesse, deren Expression in Anwesenheit oder Abwesenheit einer Tetracyclin-Verbindung reguliert werden kann, transfiziert wurde,
wobei die Tetracyclin-Verbindung Tetracyclin oder ein Tetracyclin-Analogon ist und
das Verfahren die folgenden Prozesse (a) bis (c) umfasst:
(a) vor oder nach dem folgenden Prozess (b) Knocking-out bzw. Ausschalten eines Allels einer endogenen DNA-von-Interesse-Sequenz in einer Wirtszelle, wobei die Wirtszelle eine Nalm-6-Zelle ist,
(b) Herstellen einer induzierbare DNA-von-Interesse exprimierende Zelllinie durch ein Verfahren gemäß Anspruch 7, wobei ein DNA-von-Interesse-Expressionsvektor ein Vektor ist, der als DNA-von-Interesse-Sequenz eine exogene DNA-Sequenz mit derselben Funktion wie die der endogenen DNA-von-Interesse-Sequenz der Wirtszelle umfasst, und
(c) Ausschalten (Knocking-out) des anderen Allels der endogenen DNA-von-Interesse-Sequenz in der im Prozess (b) erhaltenen Zelllinie.

9. Verfahren gemäß Anspruch 8, das außerdem den folgenden Prozess (d) nach dem Prozess (c) umfasst:
(d) Zulassen, dass die im Prozess (c) erhaltene Zelllinie die Expression der endogenen DNA-Sequenz in Anwesenheit oder Abwesenheit der Tetracyclin-Verbindung verliert,
wobei gegebenenfalls
I) die induzierbare-exprimierende Zelllinie eine Zelllinie ist, die die DNA von Interesse in Abwesenheit der Tetracyclin-Verbindung exprimiert und die die Expression der DNA von Interesse in Anwesenheit der Tetracyclin-Verbindung verliert und
im Prozess (d) die im Prozess (c) erhaltene Zelllinie die Expression der exogenen DNA-Sequenz in Anwesenheit der Tetracyclin-Verbindung verlieren gelassen wird, oder
II) die induzierbare DNA-exprimierende Zelllinie eine Zelllinie ist, die die DNA von Interesse in Anwesenheit der Tetracyclin-Verbindung exprimiert und die die Expression der DNA von Interesse in Abwesenheit der Tetracyclin-Verbindung verliert, und
im Prozess (d) die im Prozess (c) erhaltene Zelllinie die Expression der exogenen DNA-Sequenz in Abwesenheit der Tetracyclin-Verbindung verlieren gelassen wird.

10. Verfahren zur Herstellung einer veränderten DNA-Knockout-Zelllinie, in der eine endogene DNA ausgeschaltet wurde und in welche eine veränderte DNA, deren Expression in Abhängigkeit von der Anwesenheit oder Abwesenheit einer Tetracyclin-Verbindung reguliert werden kann, transfiziert wurde,
wobei die Tetracyclin-Verbindung Tetracyclin oder ein Tetracyclin-Analogon ist und
das Verfahren die folgenden Prozesse (1) bis (n) umfasst:
(l) vor oder nach dem folgenden Prozess (m) Ausschalten eines Allels einer endogenen DNA-von-Interesse-Sequenz in einer Wirtszelle, wobei die Wirtszelle eine Nalm-6-Zelle ist,
(m) Herstellen einer induzierbare DNA-exprimierenden Zelllinie durch ein Verfahren gemäß Anspruch 7,
wobei ein DNA-von-Interesse-Expressionsvektor ein Vektor ist, der als DNA-von-Interesse-Sequenz eine veränderte DNA-Sequenz der endogenen DNA-von-Interesse-Sequenz der Wirtszelle umfasst, und
(n) Ausschalten (Knocking-out) des anderen Allels der endogenen DNA-von-Interesse-Sequenz in der im Prozess (m) erhaltenen Zelllinie.

11. Verfahren gemäß Anspruch 10, wobei die veränderte DNA-Sequenz eine mutierte Sequenz der endogenen DNA-Sequenz oder eine Sequenz eines Homologons einer anderen biologischen Spezies als die der endogenen DNA-Sequenz oder eine tagfusionierte Sequenz, erhalten durch Fusionieren eines tags an die endogene DNA-Sequenz, hat.

12. Verfahren nach Anspruch 11, das außerdem den folgenden Prozess (o) nach dem Prozess (n) umfasst:
(o) Zulassen, dass die im Prozess (n) erhaltene Zelllinie die Expression der veränderten DNA-Sequenz in Anwesenheit oder Abwesenheit der Tetracyclin-Verbindung verliert,
wobei gegebenenfalls
I) die induzierbare DNA-exprimierende Zelllinie eine Zelllinie ist, die eine DNA von Interesse in Abwesenheit der Tetracyclin-Verbindung exprimiert und die die Expression der DNA von Interesse in Anwesenheit der Tetracyclin-Verbindung verliert, und
im Prozess (o) die im Prozess (n) erhaltene Zelllinie die Expression der veränderten DNA-Sequenz in Anwesenheit der Tetracyclin-Verbindung verlieren gelassen wird oder
II) die induzierbare DNA exprimierende Zelllinie eine Zelllinie ist, die eine DNA von Interesse in Gegenwart der Tetracyclin-Verbindung exprimiert und die die Expression der DNA von Interesse in Abwesenheit der Tetracyclin-Verbindung verliert, und
im Prozess (o) die im Prozess (n) erhaltene Zelllinie die Expression der veränderten DNA-Sequenz in Abwesenheit der Tetracyclin-Verbindung verlieren gelassen wird.

## Revendications

1. Procédé de recherche par criblage d'une lignée de cellules exprimant un ADN inductible qui permet l'expression d'un ADN intéressant, régulable en fonction de la présence ou de l'absence d'un composé de type tétracycline,
lequel composé de type tétracycline est la tétracycline ou un analogue de la tétracycline,
et lequel procédé comprend les étapes (A) et (B) suivantes :
A) introduire par transfection, dans une cellule hôte, un vecteur d'expression de trans-activateur et un vecteur d'expression d'ADN intéressant, étant entendu :
- que la cellule hôte est une cellule Nalm-6,
- que le vecteur d'expression de trans-activateur comprend :
une séquence polynucléotidique codant un trans-activateur et une séquence promoteur CAG qui commande la transcription de la séquence polynucléotidique codant un trans-activateur, laquelle séquence polynucléotidique codant un trans-activateur est placée sous le contrôle de la séquence promoteur CAG,
lequel trans-activateur est une protéine qui, en fonction de la présence ou de l'absence du composé de type tétracycline, se lie ou non à une séquence opérateur *tet*, et lequel trans-activateur est une protéine de fusion comprenant un répresseur Tet et un domaine d'activation de la transcription, ou une protéine de fusion comprenant un répresseur Tet inverse et un domaine d'activation de la transcription,
- et que le vecteur d'expression d'ADN intéressant comprend :
une séquence opérateur *tet*, une séquence d'ADN intéressant, une séquence promoteur qui commande la transcription de la séquence d'ADN intéressant, une séquence régulatrice bicistronique, et une séquence polynucléotidique codant un marqueur de sélection, ladite séquence régulatrice bicistronique étant placée entre la séquence d'ADN intéressant et la séquence polynucléotidique codant un marqueur de sélection, sous le contrôle de la séquence opérateur *tet* et de la séquence promoteur ;
B) et sélectionner les lignées cellulaires qui sont issues de la cellule hôte transfectée avec le vecteur d'expression de trans-activateur et le vecteur d'expression d'ADN intéressant, et dans chacune desquelles a été exprimé le marqueur de sélection présent dans le vecteur d'expression d'ADN intéressant.

2. Procédé conforme à la revendication 1, dans lequel, dans l'opération (A), c'est par co-transfection qu'on introduit le vecteur d'expression de trans-activateur et le vecteur d'expression d'ADN intéressant dans la cellule hôte.

3. Procédé conforme à la revendication 1, dans lequel
I) le trans-activateur est une protéine de fusion comprenant un répresseur Tet et un domaine d'activation de la transcription,
lequel répresseur Tet est un polypeptide qui se lie à la séquence opérateur *tet* en l'absence du composé de type tétracycline et qui ne se lie pas à la séquence opérateur *tet* en présence du composé de type tétracycline,
et dans l'étape (B), on sélectionne les lignées cellulaires qui sont issues de la cellule hôte transfectée et dans chacune desquelles le marqueur de sélection présent dans le vecteur d'expression d'ADN intéressant est exprimé en l'absence du composé de type tétracycline ;
II) ou le trans-activateur est une protéine de fusion comprenant un répresseur Tet inverse et un domaine d'activation de la transcription,
lequel répresseur Tet inverse est un polypeptide qui se lie à la séquence opérateur *tet* en présence du composé de type tétracycline et qui ne se lie pas à la séquence opérateur *tet* en l'absence du composé de type tétracycline,
et dans l'étape (B), on sélectionne les lignées cellulaires qui sont issues de la cellule hôte transfectée et dans chacune desquelles le marqueur de sélection présent dans le vecteur d'expression d'ADN intéressant est exprimé en présence du composé de type tétracycline.

4. Procédé conforme à la revendication 1, dans lequel le marqueur de sélection est un marqueur de sélection conférant une résistance à un médicament.

5. Procédé conforme à la revendication 1, qui comporte en outre l'étape (C) suivante :
C) effectuer des tests d'expression de l'ADN intéressant, en présence et en l'absence du composé de type tétracycline, sur les lignées de cellules sélectionnées dans l'opération (B).

6. Procédé conforme à la revendication 5, dans lequel l'étape (C) consiste en l'une ou l'autre des étapes (C1) et (C2) suivantes :
C1) sélectionner, parmi les lignées de cellules sélectionnées dans l'étape (B), une lignée de cellules chez laquelle l'ADN intéressant est exprimé en l'absence du composé de type tétracycline et n'est pas exprimé en présence du composé de type tétracycline ;
C2) sélectionner, parmi les lignées de cellules sélectionnées dans l'étape (B), une lignée de cellules chez laquelle l'ADN intéressant est exprimé en présence du composé de type tétracycline et n'est pas exprimé en l'absence du composé de type tétracycline.

7. Procédé de production d'une lignée de cellules exprimant un ADN intéressant inductible, qui permet l'expression d'un ADN intéressant, régulable en fonction de la présence ou de l'absence d'un composé de type tétracycline,
pour lequel la lignée de cellules exprimant un ADN intéressant inductible est obtenue par un procédé, conforme à la revendication 1, de recherche par criblage d'une lignée de cellules exprimant un ADN intéressant inductible.

8. Procédé de production d'une lignée de cellules "knock-out" à ADN inductible, dans laquelle a été introduit par transfection un ADN intéressant dont l'expression peut être régulée en fonction de la présence ou de l'absence d'un composé de type tétracycline,
lequel composé de type tétracycline est la tétracycline ou un analogue de la tétracycline,
et lequel procédé comporte les étapes (a) à (c) suivantes :
a) invalider dans une cellule hôte, avant ou après l'étape suivante (b), un seul allèle d'une séquence endogène d'ADN intéressant, laquelle cellule hôte est une cellule Nalm-6,
b) produire une lignée de cellules exprimant un ADN intéressant inductible, en opérant selon un procédé conforme à la revendication 7, dans lequel le vecteur d'expression d'ADN intéressant est un vecteur qui comporte, en tant que séquence d'ADN intéressant, une séquence d'ADN exogène qui a la même fonction que celle de la séquence endogène d'ADN intéressant de la cellule hôte,
c) et invalider, dans la lignée de cellules obtenue dans l'étape (b), l'autre allèle de la séquence endogène d'ADN intéressant.

9. Procédé conforme à la revendication 8, qui comporte en outre, après l'étape (c), l'étape (d) suivante :
d) faire en sorte que, dans la lignée de cellules obtenue dans l'étape (c), l'expression de la séquence d'ADN exogène soit perdue en présence ou en l'absence du composé de type tétracycline,
avec les options suivantes :
I) soit la lignée de cellules exprimant un ADN inductible est une lignée de cellules qui exprime l'ADN intéressant en l'absence du composé de type tétracycline et qui n'exprime pas l'ADN intéressant en présence du composé de type tétracycline,
et dans l'étape (d), on fait en sorte que, dans la lignée de cellules obtenue dans l'étape (c), l'expression de la séquence d'ADN exogène soit perdue en présence du composé de type tétracycline ;
II) soit la lignée de cellules exprimant un ADN inductible est une lignée de cellules qui exprime l'ADN intéressant en présence du composé de type tétracycline et qui n'exprime pas l'ADN intéressant en l'absence du composé de type tétracycline,
et dans l'étape (d), on fait en sorte que, dans la lignée de cellules obtenue dans l'étape (c), l'expression de la séquence d'ADN exogène soit perdue en l'absence du composé de type tétracycline.

10. Procédé de production d'une lignée de cellules "knock-out" à ADN modifié, dans laquelle un ADN endogène a été invalidé et dans laquelle a été introduit par transfection un ADN modifié dont l'expression peut être régulée en fonction de la présence ou de l'absence d'un composé de type tétracycline,
lequel composé de type tétracycline est la tétracycline ou un analogue de la tétracycline,
et lequel procédé comporte les étapes (1) à (n) suivantes :
l) invalider dans une cellule hôte, avant ou après l'étape suivante (m), un seul allèle d'une séquence endogène d'ADN intéressant, laquelle cellule hôte est une cellule Nalm-6,
m) produire une lignée de cellules exprimant un ADN inductible, en opérant selon un procédé conforme à la revendication 7, dans lequel le vecteur d'expression d'ADN intéressant est un vecteur qui comporte, en tant que séquence d'ADN intéressant, une séquence d'ADN qui est une variante modifiée de la séquence endogène d'ADN intéressant de la cellule hôte,
n) et invalider, dans la lignée de cellules obtenue dans l'étape (m), l'autre allèle de la séquence endogène d'ADN intéressant.

11. Procédé conforme à la revendication 10, dans lequel la séquence d'ADN modifiée est une variante mutée de la séquence d'ADN endogène, une séquence d'un homologue d'une espèce biologique différente de celle à laquelle appartient la séquence d'ADN endogène, ou une séquence à étiquette fusionnée, obtenue par fusion d'une étiquette à la séquence d'ADN endogène.

12. Procédé conforme à la revendication 11, qui comporte en outre, après l'étape (n), l'étape (o) suivante :
o) faire en sorte que, dans la lignée de cellules obtenue dans l'étape (n), l'expression de la séquence d'ADN modifiée soit perdue en présence ou en l'absence du composé de type tétracycline,
avec les options suivantes :
I) soit la lignée de cellules exprimant un ADN inductible est une lignée de cellules qui exprime l'ADN intéressant en l'absence du composé de type tétracycline et qui n'exprime pas l'ADN intéressant en présence du composé de type tétracycline,
et dans l'étape (o), on fait en sorte que, dans la lignée de cellules obtenue dans l'étape (n), l'expression de la séquence d'ADN modifiée soit perdue en présence du composé de type tétracycline ;
II) soit la lignée de cellules exprimant un ADN inductible est une lignée de cellules qui exprime l'ADN intéressant en présence du composé de type tétracycline et qui n'exprime pas l'ADN intéressant en l'absence du composé de type tétracycline,
et dans l'étape (o), on fait en sorte que, dans la lignée de cellules obtenue dans l'étape (n), l'expression de la séquence d'ADN modifiée soit perdue en l'absence du composé de type tétracycline.
